# EUROPEAN PATENT APPLICATION

(11) **EP 4 817 609 A1**
(43) Date of publication of application: **07.10.2026**
(21) Application number: 26181468.5
(22) Date of filing: 16.04.2021
(51) Int. Cl.: A61K 31/215, A61P 11/00, A61P 29/00, A61P 31/12, A61P 31/14

(54) **THERAPEUTIC USE OF PLEUROMUTILINS**

(30) Priority: 17.04.2020 EP 20170072; 17.04.2020 US 202063011474 P; 30.06.2020 EP 20183292
(62) Document number(s) of the earlier application(s) in accordance with Art. 76 EPC: 21723641.3 / 4 135 682
(71) Applicant: Hong Kong King-Friend Industrial Company Limited, Hong Kong (HK)
(72) Inventor: Hafner, Michael, 1030 Wien (AT); Paukner, Susanne, 1160 Wien (AT); Wicha, Wolfgang, 2460 Bruck an der Leitha (AT); Riedl, Rosemarie, 1220 Wien (AT); Ivezic-Schoenfeld, Zrinka, 2100 Korneuburg (AT)
(74) Representative: Schwarz & Partner Patentanwälte GmbH

(57) **Abstract**

A compound of formula (1) 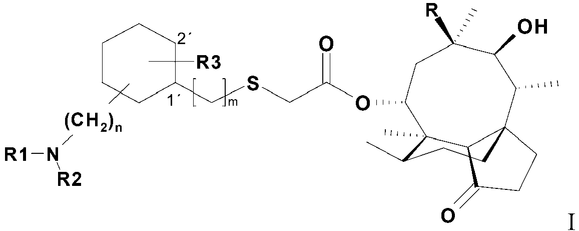 wherein
n is 0 to 4;
m is 0 or 1 with the proviso that the sulphur atom and R₃ are in vicinal position (if m = 0 then R₃ is in position 2', and if m = 1 then R₃ is on position 1');
R is ethyl or vinyl;
R₁ is hydrogen or (C₁₋₆)alkyl,
R₂ is hydrogen or
- (C₃₋₆)cycloalkyl, or
- unsubstituted (C₁₋₆)alkyl, or
- (C₁₋₆)alkyl substituted by one or more of
- hydroxy; preferably one or two,

- methoxy,
- halogen,
- (C₃₋₆)cycloalkyl, or

R₁ and R₂ together with the nitrogen atom to which they are attached form a 5 to 7 membered heterocyclic ring containing at least 1 nitrogen atom or 1 nitrogen and 1 additional heteroatom e. g. selected from N or O, or
R₁ is hydroxy and R₂ is formyl;
R₃ is OH, OR₄, a halogen atom, or
R₃ is bound to 2' and represents -O-(CH₂)ₚ-O- with p is 2 or 3;
R₄ is unsubstituted (C₁₋₆)alkyl or (C₃₋₆)cycloalkyl,
or a pharmaceutically acceptable salt, solvate, prodrug or metabolite thereof
for the specific use in the treatment or prevention of an inflammatory disease which is not mediated by bacteria.

## Description

The present invention relates to a novel therapeutic use of Pleuromutilins.

Pleuromutilin, a compound of formula 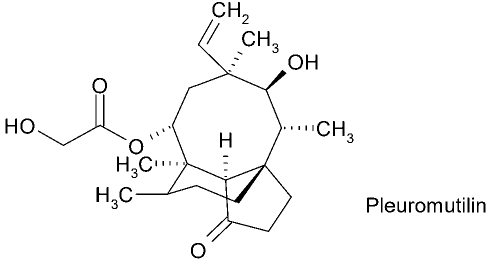 is a naturally occurring antibiotic, produced e.g. by the basidiomycetes *Pleurotus mutilus* and *P. passeckerianus,* see e.g. The Merck Index, 12th edition, item 7694.

A number of further Pleuromutilins having the principle ring structure of Pleuromutilin and being substituted at the primary hydroxy group have been developed, e.g. as antibacterials. Due to their pronounced antibacterial activity, a group of Pleuromutilin derivatives, amino-hydroxy-substituted cyclohexylsulfanylacetylmutilins, as disclosed in WO 2008/113089, have been found to be of particular interest. As described in WO 2008/113089 14-O-{[(4-Amino-2-hydroxy-cyclohexyl)-sulfanyl]-acetyl}-mutilins are particularly useful compounds because of their activity against Gram-positive and Gram-negative bacteria.

Pharmaceutical active compounds derived from Pleuromutilin (semi synthetic compounds) are inhibitors of ribosomal protein synthesis in bacteria. Representatives of semisynthetic Pleuromutilins for human use are Retapamulin (approved as AltargoP^{®}, AltabaxP^{®}), a topical agent approved for short term treatment of impetigo and infected small lacerations, abrasions or sutured wounds, and Lefamulin (approved as Xenleta^{®}) for the treatment of adults with community-acquired bacterial pneumonia (CABP). Tiamulin (Denagard^{®}) and Valnemulin (Econor^{®}) are two other semi-synthetic Pleuromutilin derivatives which have been used systemically as antibiotics in veterinary medicine for many years.

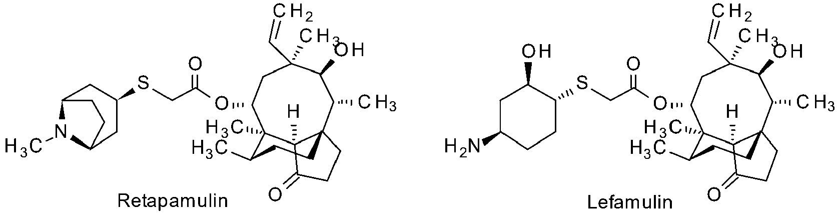

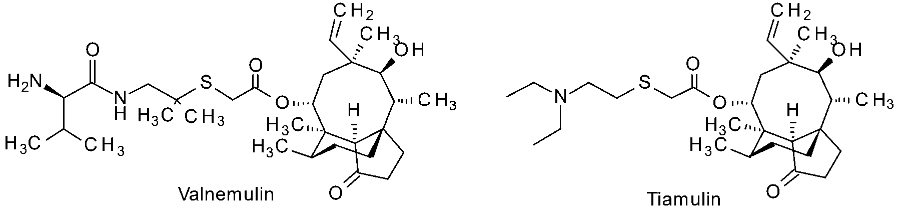

Approved semisynthetic compounds derived from Pleuromutilin have shown excellent activity against bacterial organisms which include inter alia *Streptococcus pneumoniae, Haemophilus influenzae, Staphylococcus aureus* (including MRSA), *Moraxella catarrhalis, Legionella pneumophila, Chlamydophila pneumoniae* and *Mycoplasma pneumoniae.*

Inflammatory diseases are caused by an inappropriate immune response. The inflammation may relate to various causes including for example a cell injury, ischemia, trauma, allergen, or exposure to a pathogen and when the immune system attacks the body's own tissues (autoimmune diseases). A de-regulated, e.g. over-exuberant, immune response and/or a persistent, i.e. chronic state, is considered as inflammatory disease.

In case of inappropriate reaction, the inflammatory response can be generalized involving the whole body, such as a sepsis, or primarily affect certain organs. This may result in the failure of the body's organs including e.g. the lung. For example, an acute lung injury (ALI) is defined as a syndrome of acute and persistent lung inflammation with increased vascular permeability. It is characterized by inflammatory damage to the alveolar-capillary membrane and an excessive uncontrolled inflammatory response within the lung. Acute respiratory distress syndrome (ARDS) is the most severe form of ALI and involves defective oxidation and inflammation, upregulation of adhesion molecules, increased production of cytokines and chemokines and excessive pulmonary cell apoptosis. These syndromes are associated with development of multiple organ dysfunction syndrome, which plays a pivotal role in the death of patients with multiple transfusion, shock, sepsis and ischemia-reperfusion and it remains refractory to therapy.

Chronic inflammation happens when an immune response persists, leaving the body in a constant state of alert. Over time, chronic inflammation may have a negative impact on tissues and organs.

Valnemulin has been shown to attenuate the concentration of pro-inflammatory cytokines such as TNF-α, IL-6 and IL-1β in bronchoalveolar lavage fluid and to suppress the transcription of these cytokines in lungs (Chen, Zhang et al. 2010, Inflammation 33(5):306-14), which is consistent with *in vitro* studies (Zhang, Li et al. 2009, Int Immunopharmacol 9(7-8): 810-816), and suggesting an effective scavenging of oxyradicals during the inflammatory response to lipopolysaccharide (LPS)-induced ALI and an inhibition of epithelial permeability, which may contribute to the therapeutic effect of Valnemulin. Moreover, pre-administration of Valnemulin significantly reduced the pulmonary wet-to-dry (W/D) ratio in the lung, which may attenuate the development of pulmonary edema (Chen, Zhang et al. 2010, Inflammation 33(5):306-14).

A preliminary analysis of the anti-inflammatory acitivity of Lefamulin in a lipopolysaccharide-induced lung neutrophilia model was published as preprint (Hafner, Paukner, et al. 2020, bioRxiv 2020.06.23.168393; doi: https://doi.org/10.1101/2020.06.23.168393).

Certain statements about potential antiviral and anti-inflammatory effects of Lefamulin were made in the "Q1 2020 Nabriva Therapeutics PLC Earnings Call" of May 11, 2020, (a transcript of which is available under https://www.yahoo.com/news/edited-transcript-nbrv-oq-earnings-144108621.html, downloaded June 10, 2020 as well as in a press release of May 11, 2020 (https://investors.nabriva.com/news-releases/news-release-details/nabriva-therapeutics-reports-first-quarter-2020-financial), downloaded May 28, 2020.

Viral diseases are one of the leading causes of morbidity and mortality in the world. Respiratory viruses such as influenza, respiratory syncytial virus, certain adenoviruses, rhinoviruses and corona viruses and in particular the newly emerged severe acute respiratory syndrome coronavirus 2 (SARS-CoV-2; COVID-19) have a significant impact on public health.

In Asheshov, Igor N. et. al., Antibiotics & Chemotherapy 4/4 (1954), 380-394, for the first time the antiviral activity of Pleuromutilins was described with antiviral activity of Pleuromutilin itself for an influenza A virus strain (PR8) at a concentration of 2 mg/mL. In contrast, Pleuromutilin did not show antiviral activity for polio virus in this study.

Furthermore, in Alacórn, Balbino et.al., Antiviral Research, 4 (1984), 231-243, the antiviral activity of Pleuromutilin against both, DNA and RNA viruses, in particular herpes simplex type 1 (HSV-1) virus at a test compound concentration that conferred a 50% protection of the cytopathic effect induced by HSV-1 (CPE50) of 40 µM (15 µg/mL) and activity against vesicular stomatitis virus (VSV) is described.

In WO 2009/106839 the use of Tiamulin as an antiviral agent is claimed, with effect of tiamulin on influenza A virus, porcine reproductive and respiratory syndrome virus (PRRSV) type 1 and 2 in a viral up-take assay 4 hours post inoculation with the virus at Tiamulin concentrations of 0.1-10 µg/mL compared to Valnemulin and the effect of Tiamulin on endosomal pH exemplified. Valnemulin did not exhibit antiviral activity and it was stated that other Pleuromutilin antibiotics have not been found to have an effect on viruses.

Alteration of the endosomal or lysosomal pH by Tiamulin and associated prevention of fusion of the viral membrane with endo- and lysosomes, which is a pre-requisite for viral entry, was described as potential mode-of-action.

CN 103204787B and CN 103242210 both disclose further Pleuromutilin derivatives and generally mention their use in antiviral drugs, without, however, disclosing any actual proof for an antiviral action.

### SUMMARY OF THE INVENTION

Surprisingly, it was now found that the Pleuromutilin derivatives disclosed in WO 2008/113089A1 show immunomodulatory and anti-inflammatory effects that do not relate to their antibacterial activity.

Therefore, in a first aspect the present invention relates to a compound as defined in claims 1 to 6, in particular Lefamulin, or any pharmaceutically acceptable salt, solvate, ester of metabolite thereof, for the specific use in the treatment or prevention of an inflammatory disease which is not mediated by bacteria.

In a further aspect, the present invention relates to a method of treatment or prevention of an inflammatory disease which is not mediated by bacteria, comprising administering a compound as defined in any of claims 1 to 6, in particular Lefamulin, or a pharmaceutically acceptable salt, solvate, ester of metabolite thereof to a subject in need of such treatment.

Moreover, the compounds show anti-viral activity. Thus, the compounds are also suited for use in the treatment and prevention of a disease mediated by a virus, i.e. viral infections, and in methods of treating or preventing such a disease.

Accordingly, in another aspect the present invention relates to a compound as defined in claims 1 to 6, in particular Lefamulin, or a pharmaceutically acceptable salt, solvate, prodrug or metabolite thereof, for the specific use in the treatment or prevention of a viral infection and an inflammatory condition associated with or caused by the viral infection, wherein the compound of formula I is administered both to treat and/or to prevent the viral infection itself and to treat and/or to prevent the inflammatory condition.

In a further aspect, the present invention relates to a method of treating or preventing a viral infection and an inflammatory condition associated with or caused by the viral infection, comprising administering a compound as defined in any of claims 1 to 6, in particular Lefamulin, or a pharmaceutically acceptable salt, solvate, prodrug or metabolite thereof, wherein both the viral infection and inflammatory condition are treated and/or prevented.

In yet another aspect, the present invention relates to Lefamulin in its form as acid addition salt with itaconic acid, in particular Lefamulin itaconate.

### BRIEF DESCRIPTION OF THE DRAWINGS

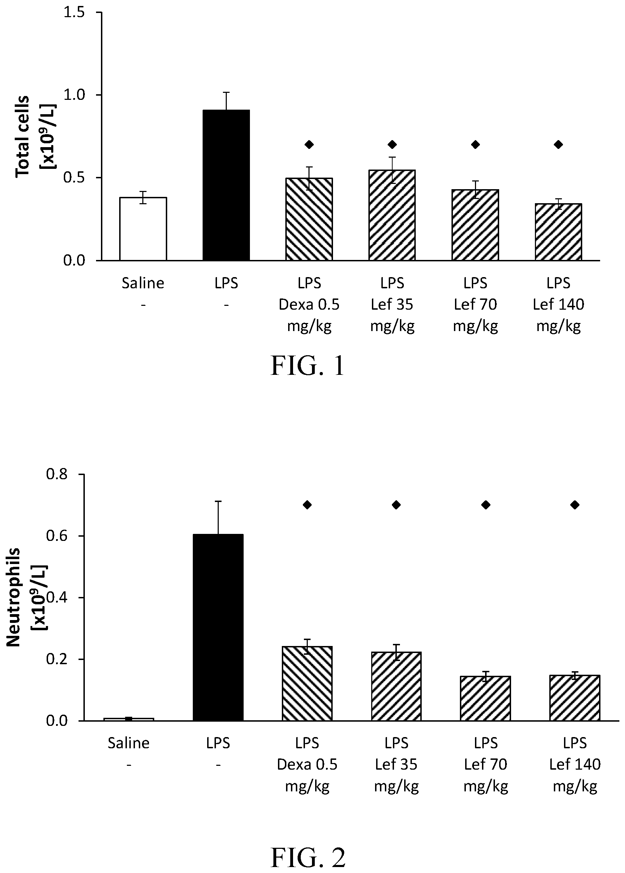

Figure 1 demonstrates the effect of Lefamulin in reducing total cell count in bronchoalveolar lavage fluid (BALF) of mice challenged with lipopolysaccharide (LPS). Error bars indicate standard error of mean. A diamond mark above the bar indicates a statistically significant decrease (p < 0.05, Mann-Whitney test) in comparison to the untreated control.
Figure 2 demonstrates the effect of Lefamulin in reducing neutrophil count in bronchoalveolar lavage fluid (BALF) of mice challenged with lipopolysaccharide (LPS). Error bars indicate standard error of mean. A diamond mark above the bar indicates a statistically significant decrease (p < 0.05, Mann-Whitney test) in comparison to the untreated control.

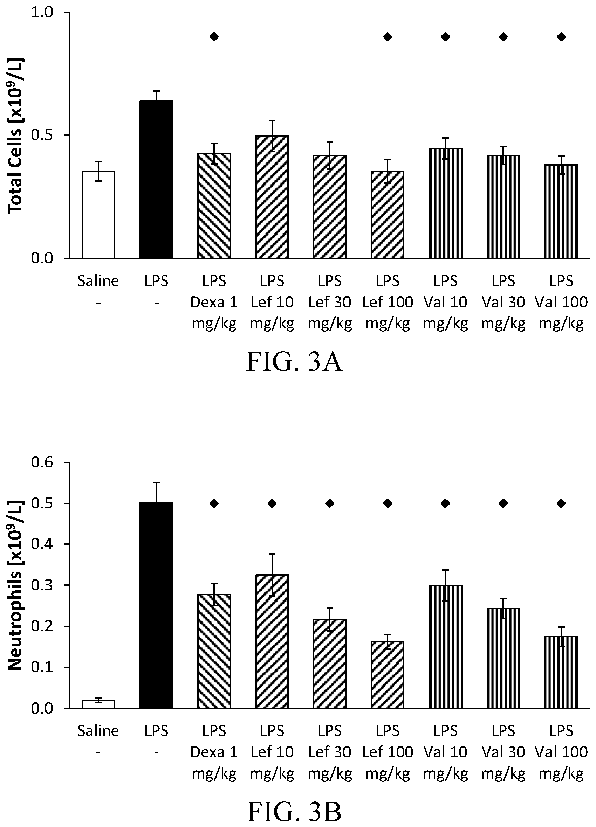

Figures 3A to 3D demonstrate the effect of Dexamethasone, Lefamulin and Valnemulin on the number of total cells (A), neutrophils (B), macrophages (C) and lymphocytes (D) in bronchoalveolar lavage fluid (BALF) of mice challenged with lipopolysaccharide (LPS). Error bars indicate standard error of mean. A diamond mark above the bar indicates a statistically significant decrease (*p* < 0.05, Mann-Whitney test) in comparison to the untreated control.

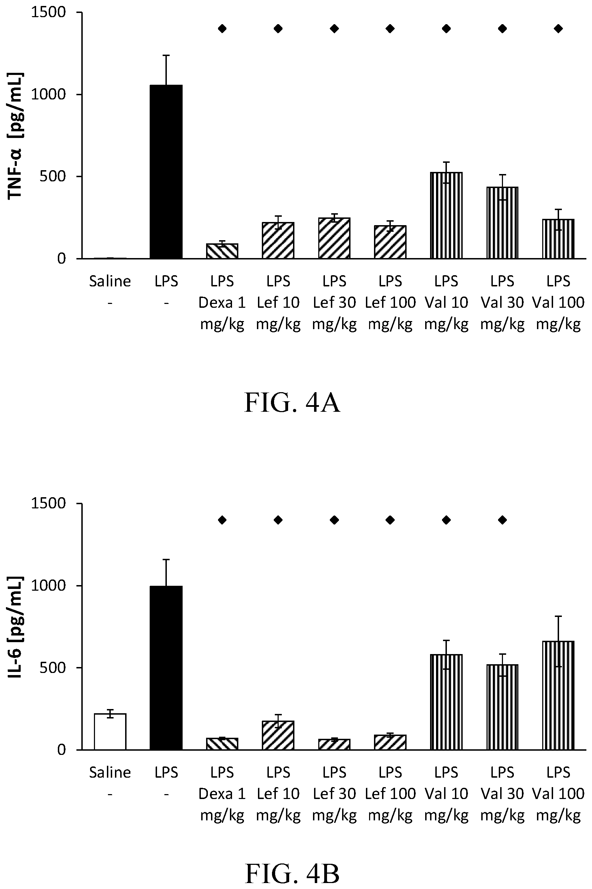

Figures 4A to 4H demonstrate the effect of Dexamethasone, Lefamulin and Valnemulin on the concentration of various markers in lung homogenate, in particular TNF-α (A), IL-6 (B), IL-1β (C), GM-CSF (D), CXCL1 (E), CXCL2 (F), CCL2 (G), and MMP9 (H). Error bars indicate standard error of mean. A diamond mark above the bar indicates a statistically significant decrease (p < 0.05, Mann-Whitney test) in comparison to the untreated control.

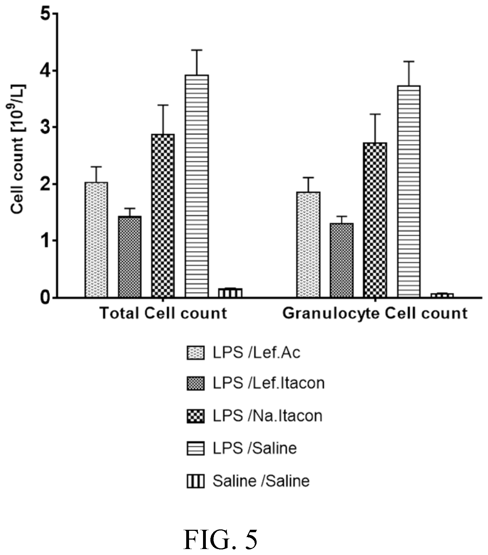

Figure 5 demonstrates the effect of sodium itaconate and Lefamulin in the form of its acetate salt and its itaconate salt in reducing cell count in bronchoalveolar lavage fluid (BALF) of mice challenged with lipopolysaccharide (LPS). Error bars indicate standard error of mean.

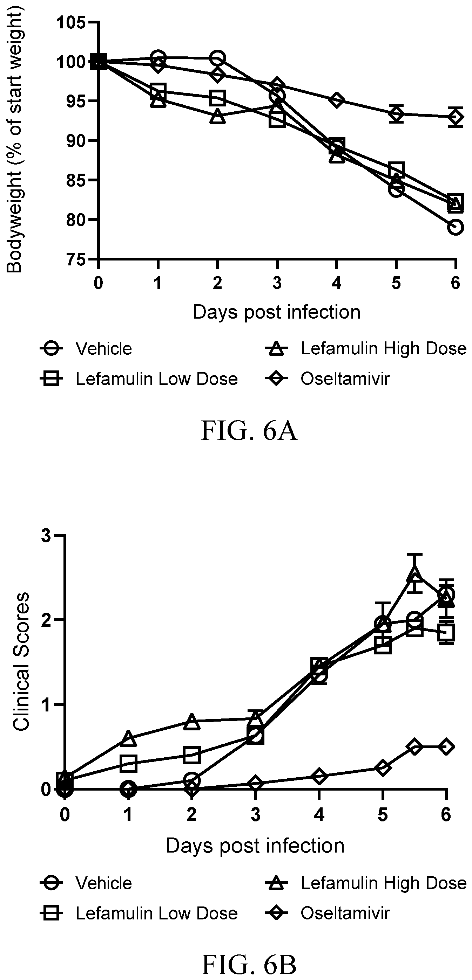

Figures 6A to 6D demonstrate the effect of Lefamulin and Oseltamivir on clinical signs in an Influenza infection mouse model, in particular on the body weight (A), clinical score (B), percentage survival (C), and lung sample histopathology scoring (D).

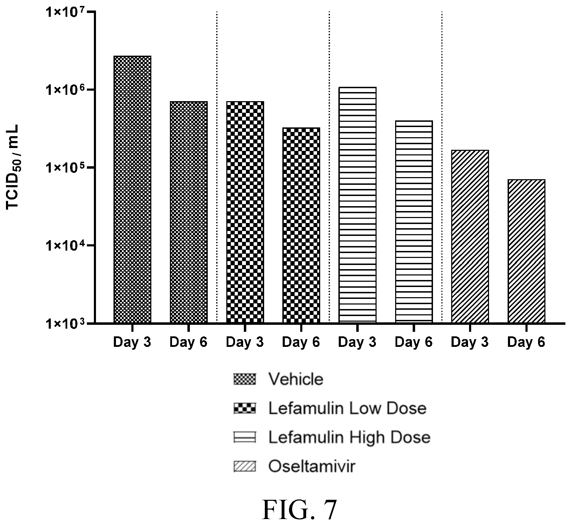

Figure 7 demonstrates the effect of Lefamulin and Oseltamivir on lung viral titer in the Influenza infection mouse model determined as 50% tissue culture infective dose (TCID₅₀) in MDCK cells.

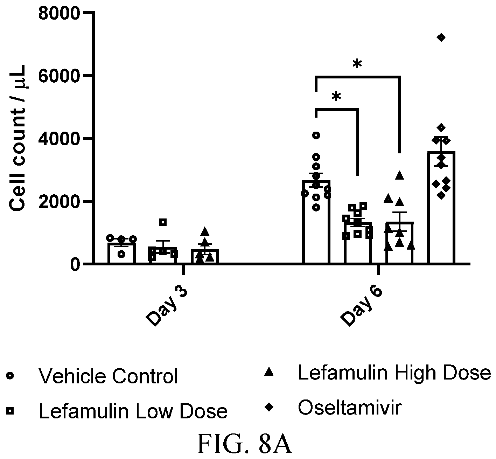

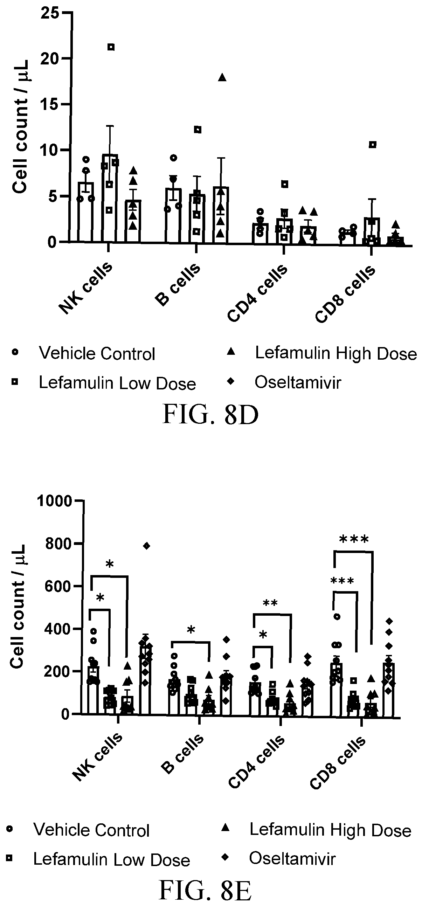

Figures 8A to 8E demonstrate the effect of Lefamulin and Oseltamivir on the number of immune cells on days 3 and 6 (A), myeloid subsets on day 3 (B), myeloid subsets (neutrophils, alveolar macrophages, macrophages) on day 6 (C), lymphocyte subsets on day 3 (D), lymphocyte subsets on day 6 (E) post-infection in the Influenza infection mouse model. Ordinary one-way ANOVA, Dunnett's multiple comparisons against the vehicle control treatment was run for each time point. *, ** and *** represent significance levels of *p* < 0.05, *p* < 0.01 and *p* < 0.001 respectively. Individual data points were excluded as outliers.

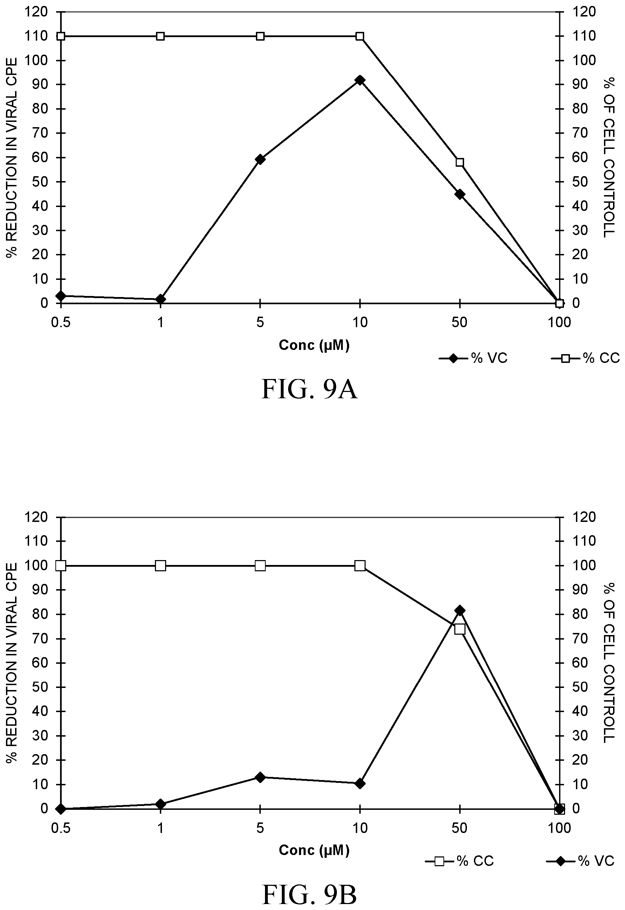

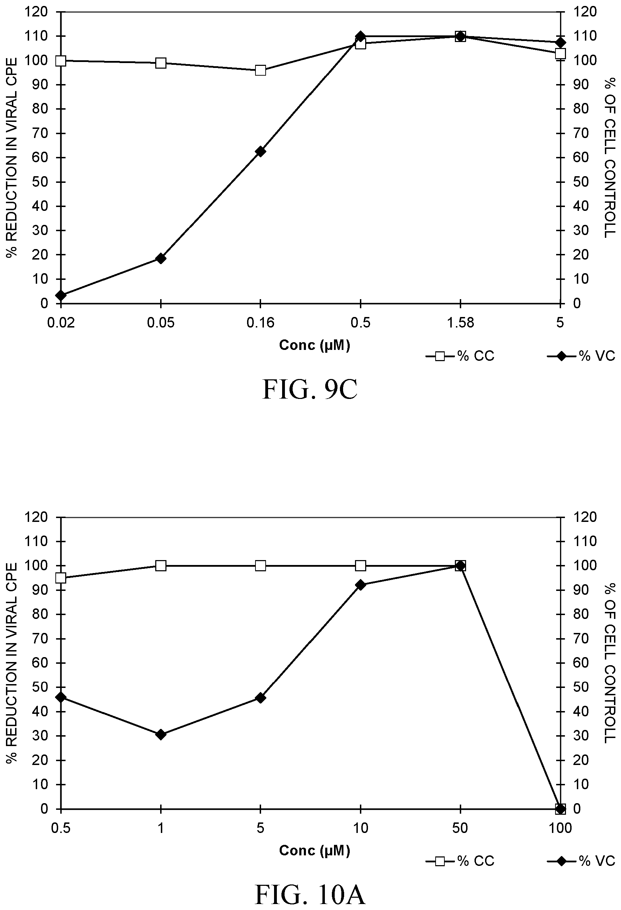

Figures 9A to 9C demonstrate the effect of Lefamulin (A), Tiamulin (B), and Remdesivir (C) against alpha corona virus 229E (HCoV-229E) in MRC-5 cells 6 days post infections with the virus.

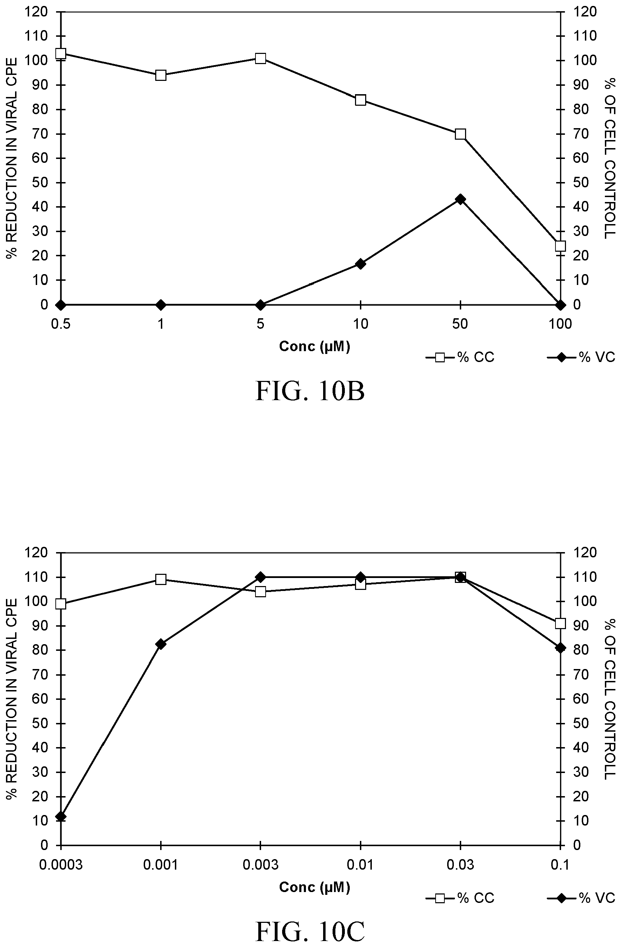

Figures 10A to 10C demonstrate the effect of Lefamulin (A), Tiamulin (B), and TMC353121 (C) against respiratory syncytial virus type A (RSV_{A2}) in HEp2 cells 6 days post infections with the virus.

### DETAILED DESCRIPTION OF THE INVENTION

Lefamulin is the INN for a compound of generic formula (I), more particular, Lefamulin is a compound of formula (VII) 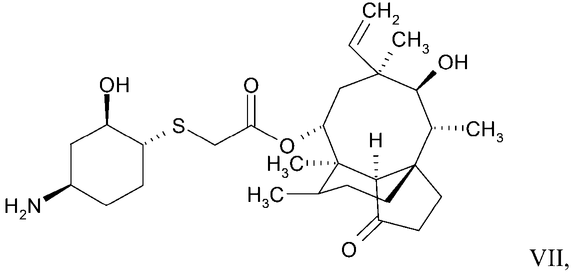 i.e. *14-O-{[(1R, 2R,* 4*R*)-4-amino-2-hydroxy-cyclohexylsulfanyl]-acetyl}-mutilin (also known as "BC-3781").

In the following, the term "Lefamulin", when generally used without additional explanation, is intended to encompass both Lefamulin in free base form, as well as its salts and solvates.

Lefamulin has been developed for systemic use to treat serious bacterial infections in humans and was approved for medical use in the United States in 2019 to treat adults with community-acquired bacterial pneumonia (CABP).

It has been found that the compounds used according to the present invention have immunomodulatory effect.

The results of the experiments show that beside antibacterial activity, Lefamulin is also immunomodulatory and has anti-inflammatory effects. As apparent from experiments using an *in vivo* model of lipopolysaccharide (LPS) induced lung inflammation in mice, Lefamulin demonstrated a significant reduction of the total cell and in particular neutrophil counts in the bronchoalveolar lavage fluid (BALF) in a dose-dependent manner, i.e. inhibiting cell infiltration. Moreover, the experiments also show a striking reduction of the concentration of proinflammatory mediators (e.g., cytokines, chemokines and other factors such as matrix metalloproteinase 9) at the site of inflammation at all doses tested.

Immune cell (e.g. neutrophil) infiltration is a multistage immunological process including release of proinflammatory mediators (e.g cytokines, chemokines and matrix metalloproteinase). These cytokines upregulate the expression of cell adhesion molecules on capillary endothelia and subsequently mediate the transmigration of neutrophils into alveolar spaces based on the chemotactic gradient. A similar mechanism is to be expected for inflammatory processes at other sites of diseases. By an alteration of immune functions (e.g. inhibition of proinflammatory mediator secretion) an inflammatory disease based on an inappropriate (over-exuberant or deregulated) or chronic inflammatory response can be treated or prevented.

Accordingly, the present invention concerns the use of the compounds as defined in claims 1 to 6, especially Lefamulin, in the treatment of an inflammatory disease, wherein the inflammatory disease is based on an inappropriate (over-exuberant or deregulated) or chronic inflammatory response. The disease is a disease which is not mediated by bacteria.

Anti-inflammatory treatment may be desired in diseases mediated by bacteria. The present invention encompasses the treatment of inflammatory diseases which are not mediated by bacteria.

According to one embodiment, the inflammatory disease may be an inflammatory disease, which is not mediated by microbes.

For the purposes of the present invention, the term "microbes" does not encompass viruses.

In a preferred embodiment of the present invention, the inflammatory disease is a result of an inappropriate (e.g. over-exuberant or de-regulated) or chronic inflammatory response, in particular the disease is a condition of over-reacting immune response, a neutrophil-dominated inflammatory disease, an auto-immune disease, an allergy, or a dermatological inflammatory disease.

In a preferred embodiment, the inflammatory disease is a condition of over-reacting immune response including for example acute lung injury (ALI) including acute respiratory distress syndrome (ARDS), sepsis and cytokine release syndrome including cytokine storm. In these conditions, the immune response is de-regulated and typically considered as being over-exuberant.

In particular, when not being mediated by bacteria, such conditions of over-reacting immune response may be a non-infectious condition (i.e. not mediated by any pathogen, including microbes and viruses) or a condition mediated by a virus.

In one embodiment, the inflammatory disease is a condition of over-reacting immune response mediated by a virus. In this embodiment, the condition of over-reacting immune response (including for example acute lung injury (ALI) including acute respiratory distress syndrome (ARDS), sepsis, cytokine release syndrome including cytokine storms) may be accompanied by a viral infection.

In a particular embodiment, the condition of over-reacting immune response is mediated by a virus, in particular a viral sepsis or an acute respiratory disease related to a viral infection such as Influenza, Severe Acute Respiratory Syndrome (SARS), Middle East Respiratory Syndrome (MERS) or COVID-19.

In another preferred embodiment, the inflammatory disease is a neutrophil-dominated inflammatory disease including for example chronic obstructive pulmonary disease (COPD), cystic fibrosis, bronchiectasis including diffuse panbronchiolitis (DPB), bronchiolitis obliterans syndrome, non-eosinophilic asthma.

In these diseases, the immune response is deregulated in terms of a domination by the activity of the neutrophil granulocytes. There is a clear medical need for treatment of neutrophil-dominated inflammatory diseases, because none of the classical anti-inflammatory therapies are effective, e.g. corticosteroids, and non-steroidal anti-inflammatory drugs do not affect neutrophil function or may even adversely prolong the neutrophil life. The results of examples 1 and 2 proof a specific effect on the neutrophil cell count for Lefamulin.

According to one embodiment of the invention, the inflammatory disease is an auto-immune disease. According to the present invention, auto-immune diseases include for example, multiple sclerosis, inflammatory bowel disease (Crohn's disease, colitis), rheumatoid arthritis, Type 1 diabetes, psoriasis, etc. These diseases are based on a de-regulated immune response as the immune response is directed to the body's own tissues. Moreover, these diseases are typically associated with chronic inflammation.

According to another embodiment of the invention, the inflammatory disease is an allergy. In allergy, the immune response is considered de-regulated as the reaction with respect to an allergen is not physiological.

In another aspect, the inflammatory disease may be a respiratory inflammatory disease or a dermatological inflammatory disease, preferably a respiratory inflammatory disease. Respiratory inflammatory diseases include for example ALI (including ARDS), Severe Acute Respiratory Syndrome (SARS), Middle East Respiratory Syndrome (MERS), COVID-19, chronic obstructive pulmonary disease (COPD), cystic fibrosis, bronchiectasis including diffuse panbronchiolitis (DPB), bronchiolitis obliterans syndrome, or non-eosinophilic asthma. Dermatological inflammatory disease include for example psoriasis, acne or rosacea.

In an embodiment of the present invention, the disease is not a bacterial pneumonia and/or not related to a bacterial pneumonia.

It has been found that the compounds used according to the present invention have both an antiviral and an immunomodulatory effects. Accordingly, the compounds are useful for a situation, wherein either an inflammatory condition is associated with a viral infection or an inflammatory condition is caused by a viral infection. In such a situation a dual effect is desired.

In one embodiment, the inflammatory condition is a condition of over-reacting immune response associated with or caused by the viral infection.

In particular, the compound is for treatment of a patient in need of both a treatment against the viral infection and a treatment against the inflammatory condition.

In a preferred embodiment, the inflammatory condition is a result of an inappropriate (e.g. over-exuberant or de-regulated) or chronic inflammatory response associated with or caused by the viral infection.

In a further embodiment, the specific use of the compound to be administered according to the present invention, in particular Lefamulin, for the treatment of a patient in need of both a treatment against the viral infection and a treatment against the inflammatory condition is provided.

In a preferred embodiment, the inflammatory condition is a condition of over-reacting immune response associated with or caused by the viral infection, including for example acute lung injury (ALI) including acute respiratory distress syndrome (ARDS), sepsis and cytokine release syndrome including cytokine storm. In these conditions, the immune response is de-regulated and typically considered as being over-exuberant.

In a particular embodiment, the specific use of the compound to be administered according to the present invention, in particular Lefamulin, for the treatment of a patient suffering from an acute respiratory syndrome related to a viral infection such as Influenza, Severe Acute Respiratory Syndrome (SARS), Middle East Respiratory Syndrome (MERS) or COVID-19, is provided.

The results of the experiments (Examples 6 to 15) show that besides its antibacterial activity, Lefamulin is also actively reducing the cytopathic effect mediated by different viruses. This antiviral effect was particularly shown for such viruses that are characterized in that they are positive- or negative sense single-stranded RNA viruses. Antiviral activity was shown for both enveloped and non-eveloped viruses, in particular several enveloped positive- or negative sense single-stranded RNA viruses (such as Coronaviridae, Paramyxoviridae, Orthomyxoviridae, and Flaviviridae). Moreover, some of the investigated viruses, including measles virus are known for a transmission involving the respiratory route, in particular airborne transmission. Corona virus and Respiratory Syncytial Virus also cause infections of the respiratory tract in humans.

In a preferred embodiment of the present invention, the viral infection is mediated by a positive- or negative-sense single-stranded RNA virus,
preferably the virus is selected from the group consisting of
- Coronaviridae including in particular human coronavirus,
- Paramyxoviridae including in particular Paramyxovirinae, such as Measles virus, and Pneumovirinae, such as Respiratory Syncytial Virus,
- Orthomyxoviridae including in particular Influenza virus,
- Flaviviridae including in particular Dengue virus and Zika virus, and
- Picornaviridae including in particular Rhinovirus.

In an other embodiment, the viral infection is an airborne disease. An airborne disease is mediated by a virus transmitted by the air.

Viral infections can affect various organs. In a preferred embodiment of the present invention, the disease is a respiratory disease, including upper and lower respiratory infections, in particular lower respiratory infections.

Treating, treatment or to treat as understood herein includes on one hand the complete curing, curation or to cure a condition (the inflammatory disease) such that it comes to its end and on the other hand also ameliorating, amelioration or to ameliorate a condition such that its symptoms are reduced at least partially or individually.

Treatment typically includes administering a compound as used according to the present invention to a subject in need thereof, e.g. in one embodiment, a subject being diagnosed to have both a viral infection as well as an inflammatory condition associated with or caused by the viral infection. The subject may have a medical history including earlier symptoms of a viral infection and later symptoms of an inflammatory condition, i.e. resulting in a viral infection being diagnosed earlier and an inflammatory condition being (co-)diagnosed later. Accordingly, in a preferred emdodiment, the compound for use according to the present invention is administered to a subject showing symptoms of or being diagnosed with both a viral infection as well as an inflammatory condition associated with or caused by the viral infection.

Preventing, prevention, or to prevent includes administering a compound before a condition is diagnosed or before onset of (all) disease symptoms of the condition.

For example, prevention according to the present invention may be considered after a subject has been infected with a virus but has not shown any symptoms of a viral infection (asymptomatic carrier), wherein a subject has been exposed and/or is prone to exposition to a virus, or wherein the subject has been diagnosed with a viral infection but not yet an inflammatory condition. Accordingly, in one embodiment, the compound of formula I is administered to treat a viral infection itself and to prevent a inflammatory condition associated with or caused by the virus.

The appropriate dosage of the compound to be administered according to the present invention, in particular Lefamulin, will, of course, vary depending upon, for example, the individual host, the mode of administration and the nature and severity of the conditions being treated. However, in general, for satisfactory results in larger mammals, for example humans, an indicated daily dosage is in the range from about 0.5 mg to 3 g of a compound used according to the present invention conveniently administered, for example, in divided doses up to four times a day.

The compound used according to the present invention may be administered by any conventional route, for example enterally, e.g. including nasal, buccal, rectal, oral administration; parenterally, e.g. including intravenous, intramuscular, subcutaneous administration; or topically, e.g. including pulmonary, epicutaneous, intranasal, intratracheal administration, e.g. in form of coated or uncoated tablets, capsules, injectable solutions or suspensions, e.g. in the form of ampoules, vials, in the form of ointments, creams, gels, pastes, inhaler powder, foams, tinctures, lip sticks, drops, sprays, or in the form of suppositories, e.g. in analogous manner to the antibiotic agent tobramycin or macrolides, such as erythromycins, e.g. clarithromycin or azithromycin.

Preferably, the compound used according to the present invention is administered via inhalation, via intravenous or subdutaneous injection, or orally.

Preferred pharmaceutical compositions of Lefamulin for injection are disclosed in WO 2016/202788 A1 the contents of which are incorporated herein by reference.

The compound used according to the present invention, in particular Lefamulin, may be administered in the form of a pharmaceutically acceptable salt, e.g. an acid addition salt, or in free form, optionally in the form of a solvate.

In one embodiment, the compound is in the form of a salt and/or a solvate.

A salt of a compound used according to the present invention includes an acid addition salt. Pharmaceutically acceptable acid addition salts include salts of a compound used according to the present invention with an acid, e.g. hydrogen fumaric acid, fumaric acid, tartaric acid, ethane-1,2-disulphonic acid, maleic acid, naphthalin-1,5-sulphonic acid, acetic acid, malic acid, lactic acid, i.e., L-lactic acid, succinic acid, salicylic acid, azelaic acid, 2-[(2,6-dichlorophenyl)amino]benzene acetic acid, hydrochloric acid, deuterochloric acid, preferably hydrochloric acid, acetic acid, L-lactic acid and maleic acid.

Of these, in the case of Lefamulin, the acetate salt of Lefamulin is especially preferred.

Preferred crystalline forms of Lefamulin as well as crystalline salt forms of Lefamulin are disclosed in WO 2011/146954 A1, the contents of which are incorporated herein by reference. Of these, the acetate salt of Lefamulin in crystalline Form B as disclosed in WO 2011/146954 A1 is especially preferred.

The present invention also provides the Lefamulin in its form as acid addition salt with itaconic acid, in particular Lefamulin itaconate. Lefamulin itaconate, i.e. compound of formula VII in the form of an itaconate salt, is disclosed herein as a new compound (Example 3). Itaconic acid can be deprotonated to the anions hydrogen itaconate and itaconate. The acid addition salt comprising Lefamulin as cation and an anion derived from itaconic acid combines the anti-inflammatory effects of Lefamulin and itaconic acid (Example 4).

In a preferred embodiment, the compound used according to the invention is Lefamulin in the form as Lefamulin acetate salt or Lefamulin itaconate salt.

The compound used according to the present invention, in particular Lefamulin, may be used for the pharmaceutical treatment contemplated herein alone or in combination with one or more other pharmaceutically active agents. Such other pharmaceutically active agents include other immune-modulating agents such as glucocorticoids, cytokines, interferons, etc. or antiviral agents. Such antiviral agents may preferably be selected from the group consisting of nucleoside and nucleotide analogues and RNA polymerase inhibitors, e.g. Remdesivir or Ribavirin, viral protease inhibitors such as Lopinavir or Ritonavir, viral neuraminidase inhibitors, such as Oseltamivir, and other agents used in antiviral therapy such as Hydroxychloroquine, interferons (interferon alfa and/or beta).

Combinations include fixed combinations, in which two or more pharmaceutically active agents are in the same formulation; kits, in which two or more pharmaceutically active agents in separate formulations are sold in the same package, e.g. with instruction for coadministration; and free combinations in which the pharmaceutically active agents are packaged separately, but instruction for simultaneous or sequential administration are given.

In another embodiment, the compound used according to the present invention, in particular Lefamulin, is the only active agent administered to the patient being diagnosed with a viral infection as well as the inflammatory conditions associated with or caused by the viral infection. I.e. the patient is treated with just one active agent to treat and/or prevent both the viral infection as well as the inflammatory condition.

A pharmaceutical composition comprising a compound used according to the present invention, in particular Lefamulin may in addition comprise at least one pharmaceutically acceptable excipient, e.g. carrier or diluent, e.g. including fillers, binders, disintegrators, flow conditioners, lubricants, sugars and sweeteners, fragrances, preservatives, stabilizers, wetting agents and/or emulsifiers, solubilizers, salts for regulating osmotic pressure and/or buffers.

Such pharmaceutical compositions may be manufactured according, e.g. analogously, to a method as conventional, e.g. by mixing, granulating, coating, dissolving, spray drying, or lyophilizing processes. Unit dosage form may contain, for example, from about 0.5 mg to about 3000 mg, such as 10 mg to about 600 mg.

A subject in need of a treatment as contemplated by the present invention may be any living subject suffering from an inflammatory disease which is not mediated by bacteria. Especially, the subject may be a human or an animal.

Particularly, the invention relates to the subject-matter and embodiments as set out below:
A. A compound of formula (I) 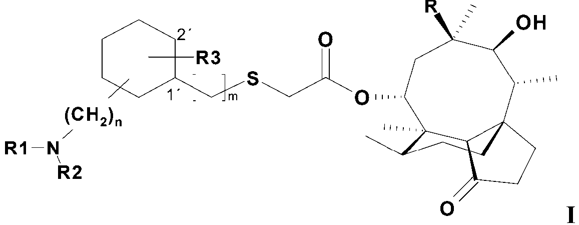 wherein
   n is 0 to 4;
   m is 0 or 1 with the proviso that the sulphur atom and R₃ are in vicinal position (if m = 0 then R₃ is in position 2', and if m = 1 then R₃ is on position 1');
   R is ethyl or vinyl;
   R₁ is hydrogen or (C₁₋₆)alkyl,
   R₂ is hydrogen or
      - (C₃₋₆)cycloalkyl, or
      - unsubstituted (C₁₋₆)alkyl, or
      - (C₁₋₆)alkyl substituted by one or more of
         - hydroxy; preferably one or two,
      - methoxy,
      - halogen,
         - (C₃₋₆)cycloalkyl, or
   R₁ and R₂ together with the nitrogen atom to which they are attached form a 5 to 7 membered heterocyclic ring containing at least 1 nitrogen atom or 1 nitrogen and 1 additional heteroatom e. g. selected from N or O, or
   R₁ is hydroxy and R₂ is formyl;
   R₃ is OH, OR₄, a halogen atom, or
   R₃ is bound to 2' and represents -O-(CH₂)ₚ-O- with p is 2 or 3;
   R₄ is unsubstituted (C₁₋₆)alkyl or (C₃₋₆)cycloalkyl,
   or a pharmaceutically acceptable salt, solvate, prodrug or metabolite thereof
   for the specific use in the treatment or prevention of an inflammatory disease which is not mediated by bacteria.
B. Compound for use according to aspect A, characterized in that the compound is selected from the group consisting of the compounds of formula (II), (III), (IV), (V) and (VI) 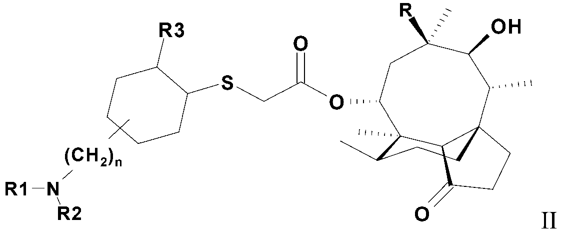 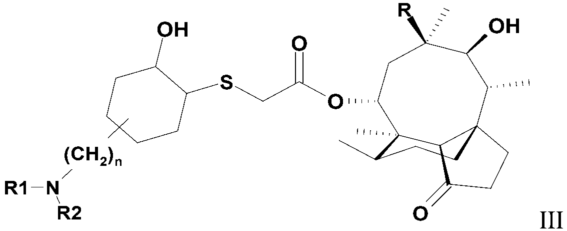 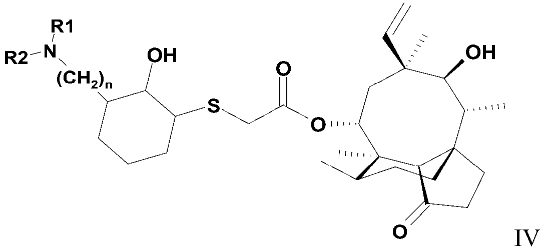 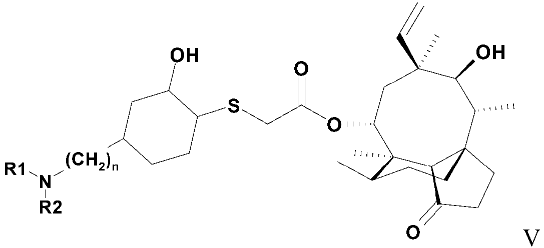 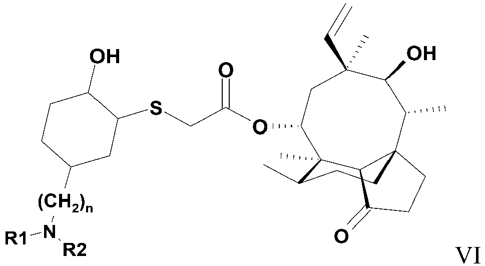 wherein in each case n, R₁ and R₂ are as defined in claim 1.
C. A compound for use according to aspects A or B, characterized in that the compound is selected from the group consisting of
   14-O-{[(1R, 2R, 4R)-4-Amino-2-hydroxy-cyclohexylsulfanyl]-acetyl}-mutilin,
   14-O-{[(1S, 2S, 4S)-4-Amino-2-hydroxy-cyclohexylsulfanyl]-acetyl}-mutilin,
   14-O-{[(1R, 2R, 5S)-5-Amino-2-hydroxy-cyclohexylsulfanyl]-acetyl}-mutilin,
   14-O-{[(1S, 2S, 5R)-5-Amino-2-hydroxy-cyclohexylsulfanyl]-acetyl}-mutilin,
   14-O-{[(1R, 2R, 4S)-4-Amino-2-hydroxy-cyclohexylsulfanyl]-acetyl}-mutilin and the (1S, 2S, 4R) diastereomer thereof,
   14-O-{[(1R, 2R, 5R)-5-Amino-2-hydroxy-cyclohexylsulfanyl]-acetyl}-mutilin,
   14-O-{[(1S, 2S, 5S)-5-Amino-2-hydroxy-cyclohexylsulfanyl]-acetyl}-mutilin,
   14-O-{[(1R, 2R, 3R)-3-Amino-2-hydroxy-cyclohexylsulfanyl]-acetyl}-mutilin and the (1S, 2S, 3S) diastereomer thereof,
   14-O-{[(1R, 2R, 4R)-4-Diethylamino-2-hydroxy-cyclohexylsulfanyl]-acetyl}-mutilin and the (1S, 2S, 4S) diastereomer thereof,
   14-O-{[(1R, 2R, 4R)-4-Ethylamino-2-hydroxy-cyclohexylsulfanyl]-acetyl}-mutilin and the (1S, 2S, 4S) diastereomer thereof,
   14-O-{[(1R, 2R, 5S)-5-Ethylamino-2-hydroxy-cyclohexylsulfanyl]-acetyl}-mutilin and the (1S, 2S, 5R) diastereomer thereof,
   14-O-{[(1R, 2R, 5S)-5-Diethylamino-2-hydroxy-cyclohexylsulfanyl]-acetyl}-mutilin and the (1S, 2S, 5R) diastereomer thereof,
   14-O-{[(1R, 2R, 4S)-4-Diethylamino-2-hydroxy-cyclohexylsulfanyl]-acetyl} -mutilin and the (1S, 2S, 4R) diastereomer thereof,
   14-O-{[(1R, 2R, 5R)-5-Diethylamino-2-hydroxy-cyclohexylsulfanyl]-acetyl}-mutilin and the (1S, 2S, 5S) diastereomer thereof,
   14-O-{[(1R, 2R, 3R)-3-Ethylamino-2-hydroxy-cyclohexylsulfanyl]-acetyl}-mutilin and the (1S, 2S, 3S) diastereomer thereof,
   14-O-{[(1R, 2R, 3R)-3-Diethylamino-2-hydroxy-cyclohexylsulfanyl]-acetyl}-mutilin and the (1S, 2S, 3S) diastereomer thereof,
   14-O-{[(1R, 2R, 4S)-4-(Formyl-hydroxy-amino)-2-hydroxy-cyclohexylsulfanyl]-acetyl}-mutilin and the (1S, 2S, 4R) diastereomer thereof,
   14-O-{[(1R, 2R, 5S)-5-(Formyl-hydroxy-amino)-2-hydroxy-cyclohexylsulfanyl]-acetyl}-mutilin and the (1S, 2S, 5R) diastereomer thereof,
   14-O-{[(1R, 2R, 3R/S)-3-(Formyl-hydroxy-amino)-2-hydroxy-cyclohexylsulfanyl]-acetyl}-mutilin and the (1S, 2S, 3R/S) diastereomer thereof,
   14-O-{[(1R, 2R, 5S)- 2-Hydroxy-5-methylamino-cyclohexylsulfanyl]-acetyl}-mutilin and the (1S, 2S, 5R) diastereomer thereof,
   14-O-{[(1R, 2R, 5S)-5-Allylamino-2-hydroxy-cyclohexylsulfanyl]-acetyl}-mutilin and the (1S, 2S, 5R) diastereomer thereof,
   14-O-{[(1R, 2R, 5S)-2-Hydroxy-5-(2-methoxy-ethylamino)-cyclohexylsulfanyl]-acetyl}-mutilin and the (1S, 2S, 5R) diastereomer thereof,
   14-O-{[(1R, 2R, 4R*)-2-Hydroxy-4-(2-hydroxy-ethylamino)-cyclohexylsulfanyl]-acetyl}-mutilin and the (1S, 2S, 4S*) diastereomer thereof,
   14-O-{[(1R, 2R, 4R*)-4-Cyclohexylamino-2-hydroxy-cyclohexylsulfanyl]-acetyl}-mutilin and the (1S, 2S, 4S*) diastereomer thereof,
   14-O-{[(1R, 2R, 4R*)-4-Cyclopropylamino-2-hydroxy-cyclohexylsulfanyl]-acetyl}-mutilin and the (1S, 2S, 4S*) diastereomer thereof,
   14-O-{[(1R, 2R, 5S*)-4-Cyclopropylamino-2-hydroxy-cyclohexylsulfanyl]-acetyl}-mutilin and the (1S, 2S, 5R*) diastereomer thereof,
   14-O-{[(1R, 2R, 4S*)-4-Cyclopropylamino-2-hydroxy-cyclohexylsulfanyl]-acetyl}-mutilin and the (1S, 2S, 4R*) diastereomer thereof,
   14-O-{[(1R, 2R, 5R*)-2-Hydroxy-5-morpholin-4-yl-cyclohexylsulfanyl]-acetyl}-mutilin and the (1S, 2S, 5S*) diastereomer thereof,
   14-O-{[(1R, 2R, 5S*)-2-Hydroxy-5-morpholin-4-yl-cyclohexylsulfanyl]-acetyl}-mutilin and the (1S, 2S, 5R*) diastereomer thereof,
   14-O-{[(1R, 2R, 5S)-5-Amino-2-hydroxy-cyclohexylsulfanyl]-acetyl }-19,20-dihydro-mutilin and the (1S, 2S, 5R) diastereomer thereof,
   14-O-{[(1R, 2R, 5S)-5-Ethylamino-2-hydroxy-cyclohexylsulfanyl]-acetyl}-19,20-dihydro-mutilin and the (1S, 2S, 5R) diastereomer thereof,
   14-O-{[(1R, 2R, 5R)-5-Amino-2-hydroxy-cyclohexylsulfanyl]-acetyl}-19,20-dihydro-mutilin and the (1S, 2S, 5S) diastereomer thereof,
   14-O-{[(1R, 2R)-4-Aminomethyl-2-hydroxy-cyclohexylsulfanyl]-acetyl}-mutilin and the (1S, 2S) diastereomers thereof,
   14-O-{[5-Amino-2-chloro-cyclohexylsulfanyl]-acetyl}-mutilin,
   14-O-{[4-Amino-2-chloro-cyclohexylsulfanyl]-acetyl}-mutilin,
   14-O-[(4-Amino-1-hydroxy-cyclohexylmethylsulfanyl)-acetyl]-mutilin,
   14-O-{[(1R, 2R)-2-Hydroxy-5-(3-methylamino-propyl)-cyclohexylsulfanyl]-acetyl}-mutilin and the (1S, 2S) diastereomer thereof,
   14-O-{[(1R, 2R)-2-Hydroxy-4-(3-methylamino-propyl)-cyclohexylsulfanyl]-acetyl}-mutilin and the (1S, 2S) diastereomer thereof,
   14-O-{[(1R, 2R)-5-(3-Amino-propyl)-2-hydroxy-cyclohexylsulfanyl]-acetyl}-mutilin and the (1S, 2S) diastereomer thereof,
   14-O-{[(1R, 2R)-4-(3-Amino-propyl)-2-hydroxy-cyclohexylsulfanyl]-acetyl}-mutilin and the (1S, 2S) diastereomer thereof,
   14-O-{[(6R, 8R)-8-Amino-1,4-dioxa-spiro[4.5]dec-6-ylsulfanyl]-acetyl}-mutilin and the (6S, 8S) diastereomer thereof,
   14-O-{[4-Amino-2-methoxy-cyclohexylsulfanyl]-acetyl}-mutilin,
      and,
   14-O-{[5-Amino-2-methoxy-cyclohexylsulfanyl]-acetyl}-mutilin.
D. A compound for use according to any of aspects A to C, characterized in that the compound is Lefamulin.
E. A compound for use according to any of aspects A to D, characterized in that the compound is in the form of a salt and/or a solvate.
F. A compound for use according to any of aspects A to E, characterized in that the compound is Lefamulin in the form as Lefamulin acetate salt or Lefamulin itaconate salt.
G. A compound for use according to any of the preceding aspects, characterized in that the inflammatory disease is a result of an inappropriate or chronic inflammatory response.
H. A compound for use according to any of of aspects A to G, characterized in that the inflammatory disease is a condition of over-reacting immune response, a neutrophil-dominated inflammatory disease, an auto-immune disease, an allergy, or a dermatological inflammatory disease, in particular a condition of over-reacting immune response or a neutrophil-dominated inflammatory disease.
I. A compound for use according to aspect H, wherein the inflammatory disease is a condition of over-reacting immune response selected from the group consisting of acute lung injury (ALI) including acute respiratory distress syndrome (ARDS), sepsis, and cytokine release syndrome including cytokine storm.
J. A compound for use according to aspect I, wherein the inflammatory disease is a neutrophil-dominated inflammatory disease selected from the group consisting of chronic obstructive pulmonary disease (COPD), cystic fibrosis, bronchiectasis including diffuse panbronchiolitis (DPB), bronchiolitis obliterans syndrome, non-eosinophilic asthma.
K. A compound for use according to aspects H or I, wherein the inflammatory disease is a condition of over-reacting immune response mediated by a virus, in particular a viral sepsis or an acute respiratory disease related to a viral infection such as Influenza, Severe Acute Respiratory Syndrome (SARS), Middle East Respiratory Syndrome (MERS) or COVID-19.
L. A compound for use according to any of claims A to K, characterized in that the inflammatory disease is an inflammatory disease which is not mediated by microbes.
M. A compound for use according to any of the preceding aspects, characterized in that the inflammatory disease is a condition associated with or caused by a viral infection, wherein the compound of formula I is administered both to treat and/or to prevent the viral infection itself and to treat and/or to prevent the inflammatory condition.
N. A compound for use according to aspect M, characterized in that the inflammatory condition is a result of an inappropriate or chronic inflammatory response associated with or caused by the viral infection.
O. A compound for use according to aspect M, characterized in that the inflammatory condition is a condition of over-reacting immune response associated with or caused by the viral infection.
P. A compound for use according to any of the aspects M to O for treatment of a patient in need of both a treatment against the viral infection and a treatment against the inflammatory condition.
Q. A compound for use according any of the aspects M to P, characterized in that the viral infection is mediated by a positive- or negative-sense single-stranded RNA virus, preferably the virus is selected from the group consisting of
   - Coronaviridae including in particular human coronavirus,
   - Paramyxoviridae including in particular Paramyxovirinae, such as Measles virus, and Pneumovirinae, such as Respiratory Syncytial Virus,
   - Orthomyxoviridae including in particular Influenza virus,
   - Flaviviridae including in particular Dengue virus and Zika virus, and
   - Picornaviridae including in particular Rhinovirus.
R. A compound for use according any of the aspects M to Q, characterized in that the viral infection is an airborne disease.
S. A compound for use according to any of the preceding aspects, characterized in that the viral infection is a respiratory disease.
T. A method of treatment or prevention of an inflammatory disease which is not mediated by bacteria comprising administering a compound as defined in any of aspects A to F, in particular Lefamulin, or a pharmaceutically acceptable salt, solvate, ester of metabolite thereof to a subject in need of such treatment.
U. A method according to aspect T, wherein the inflammatory disease is a result of an inappropriate or chronic inflammatory response.
V. A method according to aspects T or U, wherein the inflammatory disease is a condition of over-reacting immune response, a neutrophil-dominated inflammatory disease, an auto-immune disease, an allergy, or a dermatological inflammatory disease, in particular a condition of over-reacting immune response or a neutrophil-dominated inflammatory disease.
W. A method according to claim V, wherein the inflammatory disease is a condition of over-reacting immune response selected from the group consisting of acute lung injury (ALI) including acute respiratory distress syndrome (ARDS), sepsis, and cytokine release syndrome including cytokine storm.
X. A method according to claim V, wherein the inflammatory disease is a neutrophil-dominated inflammatory disease selected from the group consisting of chronic obstructive pulmonary disease (COPD), cystic fibrosis, bronchiectasis including diffuse panbronchiolitis (DPB), bronchiolitis obliterans syndrome, non-eosinophilic asthma.
Y. A method according to claims V or W, wherein the condition of over-reacting immune response mediated by a virus, in particular a viral sepsis or an acute respiratory disease related to a viral infection such as Influenza, Severe Acute Respiratory Syndrome (SARS), Middle East Respiratory Syndrome (MERS) or COVID-19.
Z. A method according to claims T to Y, wherein the inflammatory disease in that the inflammatory disease is an inflammatory disease which is not mediated by microbes.
AA. A method of treating or preventing according to any of claims T to Z, wherein the inflammatory disease is an inflammatory condition associated with or caused by a viral infection, is administered both to treat and/or to prevent the viral infection itself and to treat and/or to prevent the inflammatory condition.
AB. A method according to claim AA, wherein the inflammatory condition is a result of an inappropriate or chronic inflammatory response associated with or caused by the viral infection.
AC. A method according claim AA, characterized in that the inflammatory condition is a condition of over-reacting immune response associated with or caused by the viral infection.
AD. A method according to any of claims AA to AC, for treatment of a patient in need of both a treatment against the viral infection and a treatment against the inflammatory condition.
AE. A method of treatment according to any one of claims AA to AD, wherein the viral infection is mediated by a positive- or negative-sense single-stranded RNA virus, preferably the virus is selected from the group consisting of
   - Coronaviridae including in particular human coronavirus,
   - Paramyxoviridae including in particular Paramyxovirinae, such as Measles virus, and Pneumovirinae, such as Respiratory Syncytial Virus,
   - Orthomyxoviridae including in particular Influenza virus,
   - Flaviviridae including in particular Dengue virus and Zika virus, and
   - Picornaviridae including in particular Rhinovirus.
AF. A method of treatment according to any one of claims AA to AE, wherein the viral infection is an airborne disease.
AG. A method of treatment according to any one of claims AA to AF, wherein the viral disease is a respiratory disease.
AH. Lefamulin in its form as acid addition salt with itaconic acid, in particular Lefamulin itaconate.
AI. Lefamulin or a pharmaceutically acceptable salt, solvate, or prodrug thereof for the specific use in the treatment or prevention of an inflammatory disease.
AJ. Lefamulin or a pharmaceutically acceptable salt, solvate, or prodrug thereof for use in the treatment or prevention of a disease, wherein the inflammatory disease is characterized by neutrophilic inflammation.
AK. Lefamulin or a pharmaceutically acceptable salt, solvate, or prodrug thereof for use in the treatment or prevention of an inflammatory disease, wherein the inflammatory disease is a neutrophil-dominated inflammatory disease or a neutrophilic inflammation.

### Examples

Herein, including the examples, the following abbreviations are used:
- ¹H-NMR: proton nuclear magnetic resonance spectroscopy
- °C: degrees Celsius
- µM: micromolar concentration
- ALI: acute lung injury
- ARDS: acute respiratory distress syndrome
- BALB/c: laboratory-bred mouse strain
- BALF: bronchoalveolar lavage fluid
- BC-3781: Lefamulin
- CC: Cell Control
- CCL2: chemokine (C-C motif) ligand 2
- CoV: corona virus
- CPE: cytopathic effects, in particular virus-induced
- CXCL1: chemokine (C-X-C motif) ligand 1
- CXCL2: chemokine (C-X-C motif) ligand 2
- DMEM: Dulbecco's modified Eagle's medium
- DMF: N,N-dimethylformamide
- DMSO: dimethylsulfoxide
- eq: equivalents
- EC₅₀: Half maximal (fifty-percent) effective concentration
- FBS: Fetal bovine serum
- GM-CSF: Granulocyte-macrophage colony-stimulating factor
- HeLa: immortal human epithelial cell line
- HEp2: human epithelial cell line
- Huh7: human liver cell line
- IL-1β: interleukin 1 beta
- IL-6: interleukin 6 (IL-6)
- IN: intranasal
- IP: intraperitoneal
- LPS: lipopolysaccharide
- MDCK: Madin-Darby Canine Kidney cells
- MMP9: matrix metallopeptidase 9
- MOI: Multiplicity of infection
- MRC-5: Medical Research Council cell strain 5
- M: molarity
- MS: mass spectrometry
- m/z: mass/charge ratio
- MTBE: methyl-tert-butylether
- nm: nanometer
- PBS: phosphate-buffered saline
- PO: per os
- SC: subcutaneous
- TC₅₀: Half maximal (fifty-percent) toxic concentration
- TCID₅₀: Fifty-percent (half maximal) tissue culture infective dose
- TNF-α: tumor necrosis factor alpha
- VC: reduction in viral CPE
- XTT: 2,3-bis-(2-methoxy-4-nitro-5-sulfophenyl)-2H-tetrazolium-5-carboxanilide

### Example 1

**Objective:** The anti-inflammatory effects of Lefamulin were investigated in a model of LPS induced lung neutrophilia by determination of total cell count and neutrophil count in the BALF at 4 hours post LPS administration.

**Methodology:** Six groups of animals were tested, each consisting of 8 BALB/c mice. Compound Lefamulin was tested at subcutaneous doses of 35, 70 and 140 mg/kg, administered half an hour prior to the intranasal LPS challenge. Reference compound Dexamethasone, a known and clinically used anti-inflammatory drug, was orally administered one hour prior LPS challenge at dose of 0.5 mg/kg. All mice were anaesthetized prior LPS challenge, with a mixture of ketamine hydrochloride and xylazine hydrochloride injected intraperitoneally. Healthy control mice received 50 µL of saline, and all other animals received 5 µg LPS from *E. coli*/50 µL saline intranasally, to induce pulmonary neutrophilia. Approximately 4 hrs after LPS administration, mice were euthanized by an intraperitoneal overdose of anaesthetics. Tracheostomy was performed and a tracheal catheter was clamped into the trachea. The lungs were washed three times with cold PBS in a total volume of 1 mL (0.4, 0.3 and 0.3 mL). Collected BALF were placed into the Eppendorf test tubes and centrifuged in a desktop Eppendorf centrifuge (5 min/ 3500 rpm/4°C). Obtained cell plugs were re-suspended in 600 µL of PBS by vortexing. BALF was immediately analysed for total and differential cell counts on an automated haematology analyser Sysmex XT-2000iV. Statistical analyses were performed using GraphPad Prism version 5.04 (GraphPad Software, Inc., La Jolla, CA, USA). Between-group differences were determined using the Mann-Whitney test and were considered statistically significant when P<0.05.

### Results:

The results of the BALF analyses (total and neutrophil cell counts) four hours following intranasal LPS challenge are presented in Figures 1-2. LPS challenge induced a statistically significant increase in total cell and neutrophil count, as compared to saline challenged controls. Compared with LPS challenged animals, mice pre-treated with reference substance Dexamethasone PO one hour prior challenge, showed statistically significant reduction of both, total and neutrophil cell counts in BALF. There was also statistically significant reduction in total and neutrophil cell counts in all groups treated with Lefamulin at the indicated doses (p<0.05 vs lipopolysaccharide challenge), in a dose dependent manner. Almost complete impairment of cell influx was observed at the highest dose of 140 mg/kg. Lefamulin-associated reductions in BALF cell counts were comparable at all Lefamulin doses to those seen following treatment with the reference substance Dexamethasone, a known anti-inflammatory drug. Of note, exposure to 70 mg/kg Lefamulin SC per day corresponds to the exposure achieved in humans when administering the approved clinical daily dosages of 2 x 600 mg oral or 2 x 150 mg intravenously for the treatment of bacterial pneumonia.

Inhibition of neutrophilic lung infiltration may be beneficial, as in the early phase of ARDS, there is widespread neutrophilic alveolitis with disruption of the alveolar epithelial and endothelial barriers, which leads to the formation of protein-rich edema in the interstitium and alveolar spaces (Matthay, Ware et al. 2012, Clin Invest 122(8): 2731-2740.).

### Example 2

**Objective:** In a second study comparable to the above-described, the activity of Lefamulin was further investigated and compared with Valnemulin at doses of 10, 30 and 100 mg/kg in an LPS induced lung neutrophilia model in mice including evaluation of cytokine and chemokine concentrations in addition to determination of cell counts in BALF.

**Methodology:** Prior to intranasal (IN) LPS administration, mice were anaesthetized intraperitoneally (IP) with a combination of ketamine and xylazine. For IN instillation mice were held in a tilted supine position with their heads elevated to between 60 and 75 degrees above their feet during and after (for approximately 1 minute) instillation. Challenge was performed 30 minutes post treatment with 5 µg of LPS in 50 µL of saline/mouse. Unchallenged control mice received 50 µL of saline. The groups size was 8 animals per group.

Half an hour before LPS challenge, 1 mg/kg of reference compound Dexamethasone (positive control) was administered intraperitoneally (IP). Dexamethasone was dissolved in 0.5% methylcellulose in water. Application volume was 10 mL/kg. 10, 30, or 100 mg/kg Lefamulin or Valnemulin were administered subcutaneously (SC) between shoulders, 30 minutes before the LPS challenge. Lefamulin and Valnemulin were dissolved in 0.9% saline solution. 0.9% saline solution was used as a vehicle control. Application volume was 10 mL/kg. Four hours post LPS challenge, mice were sacrificed by ketamine (200 mg/kg) and xylazine (16 mg/kg) overdosing, after which BALF and lung tissue were sampled.

In order to collect bronchoalveolar lavage fluid (BALF) a cannula was inserted into trachea, after which lungs were lavaged with 3 volumes of PBS (0.4, 0.3 and 0.3 mL, total 1 mL). Collected BALF was placed into the Eppendorf test tube, centrifuged in desktop Eppendorf centrifuge (5 min/1303xg/4°C), and cells' plugs were re-suspended in 600 µL of PBS by shaking closed tubes content on vortex. Re-suspended BAL cells were immediately analyzed for total and differential cell count on automated haematology analyser Sysmex XT-2000iV.

For determination of certain markers, lung tissue samples were taken. The lungs were exposed and excised by gently opening the thorax and by cutting down either side of the sternum and ribs and trimming back. Lungs were removed and placed into pre-weighed sterile Precellys. Post-sampling, Precellys test tubes were weighed once again, snap frozen and placed onto -80°C until analyses. Tissue was homogenized.

Concentrations of CCL2, CXCL1, CXCL2, GM-CSF, IL-6, and TNF-α were determined using Mouse Premixed Multi-Analyte Kit following manufacturer's protocol (R&D Systems). Standard dilution series and samples were prepared in parallel and incubated with a biotin antibody cocktail. The concentration was determined using the respective streptavidin-phycoerythrin conjugate and read-out with a Luminex 200 instrument. Concentrations of these markers were determined by interpolation from standard curves by XPONENT software (Applied Cytometry). Measured values (MFI) were blank corrected, with standard curve fit five parameters logistic.

For analysis of MMP-9 and IL-1β, whole mice lungs were thawed from -80°C and homogenized in 1 mL of PBS supplemented with protease inhibitors in Precellys CK28 Hard Tissue tubes using Precellys instrument and program: shaking at 6800 rpm for 30 sec with 15 sec pauses, repeated 3 times. After homogenization, samples were centrifuged for 10 min at 18000 × g, 4°C and supernatants were collected for analysis. Concentrations of MMP-9 and IL-1β were determined using Mouse DuoSet ELISA kit following manufacturer's protocol (R&D Systems). Standard dilution series and samples were processed in parallel using plates prepared with the MMP-9 or IL-1β capture antibodies. The concentration was determined using the respective detection antibody and reagents for read-out. Absorbance was measured at 450 nm using SpectraMax i3 instrument. Concentrations of MMP9 and IL-1β in samples were determined by interpolation from standard curve.

Statistical analysis was performed using GraphPad Prism software (version 8.1.1). Outliers were identified using Grubbs test. For the evaluation of the total and differential cell counts, and marker concentrations non-parametric statistics (Mann-Whitney test) was performed. Differences between treated groups vs. vehicle group were considered statistically significant at p<0.05.

### Results:

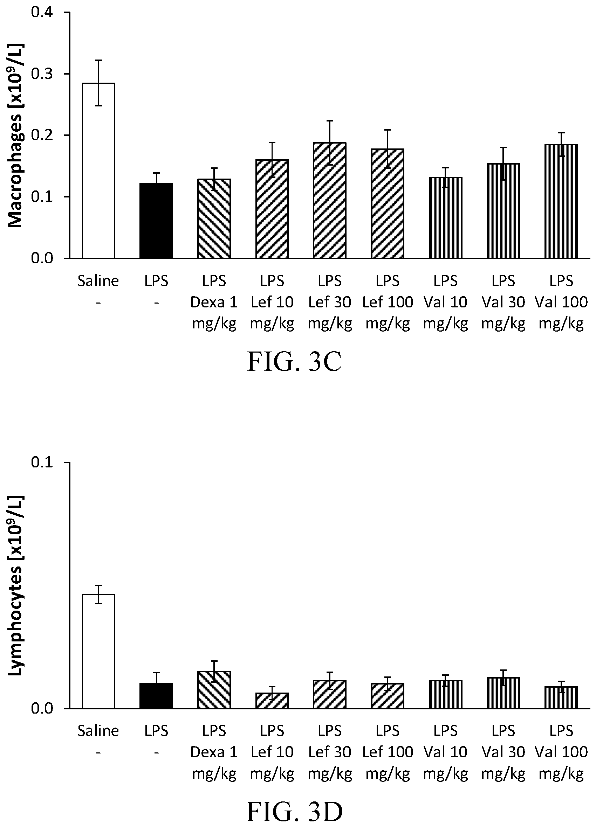

The results of the BALF analyses (cell counts) are presented in Figures 3A to 3D. LPS challenge induced a significant increase in total cell and neutrophil count, as compared to saline challenged controls. Subcutaneous pre-treatment with 10, 30, or 100 mg/kg Lefamulin reduced dose-dependently total cell counts in BALF (Fig. 3A). The changes are statistically significant at 100 mg/kg (p<0.05). As in the results of the previous study, the effect was comparable to the anti-inflammatory drug Dexamethasone (positive control). A dose-dependent and significant reduction for all investigated concentrations of Lefamulin was observed for the neutrophil cells (Fig. 3B). In contrast, the macrophage and lymphocyte cell counts were not significantly affected by Lefamulin or Valnemulin (Fig. 3C and 3D).

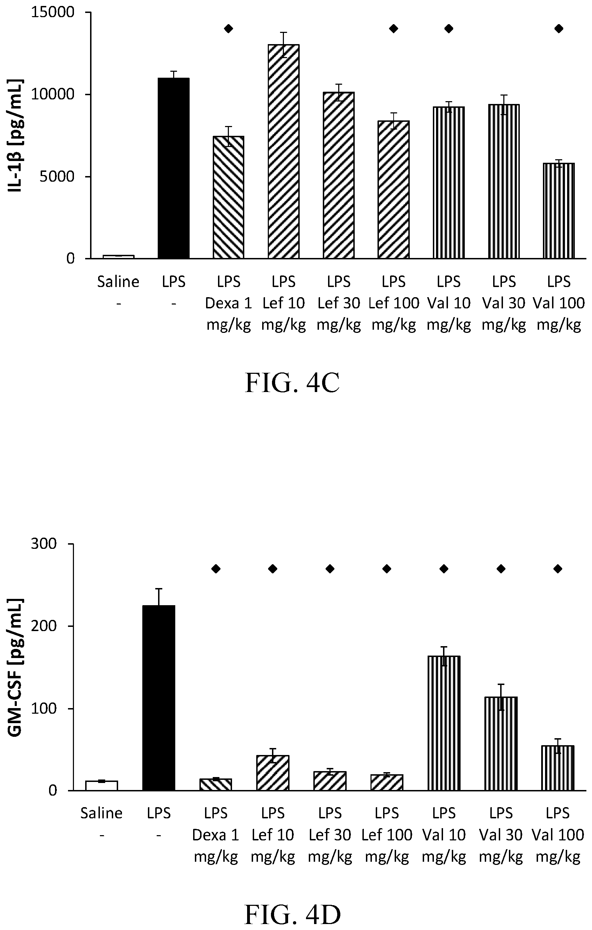

The pro-inflammatory cytokines tumor necrosis factor alpha (TNF-α), interleukin 6 (IL-6), and interleukin 1 beta (IL-1β) are increased in the lung homogenate after LPS-challenge in the mouse model in comparison to the saline control (Fig. 4A to 4C). Subcutaneous pretreatment with 10, 30, or 100 mg/kg Lefamulin reduced TNF-α and IL-6 levels by 50 % or more (Fig. 4A and 4B), whereas Valnemulin was less active at when compared with the same doses. For IL-1β subcutaneous pre-treatment with 100 mg/kg Lefamulin significantly reduced IL-1β level in lung homogenate by 24% (Fig. 4C).

Granulocyte-macrophage colony-stimulating factor (GM-CSF), a cytokine known to influence cell migration, was increased in the LPS-induced mouse model (Fig. 4D). Lefamulin almost completely inhibited GM-CSF in lung homogenate. Valnemulin also showed a reduction of GM-CSF levels, but was less active than Lefamulin at all doses tested (Fig. 4D).

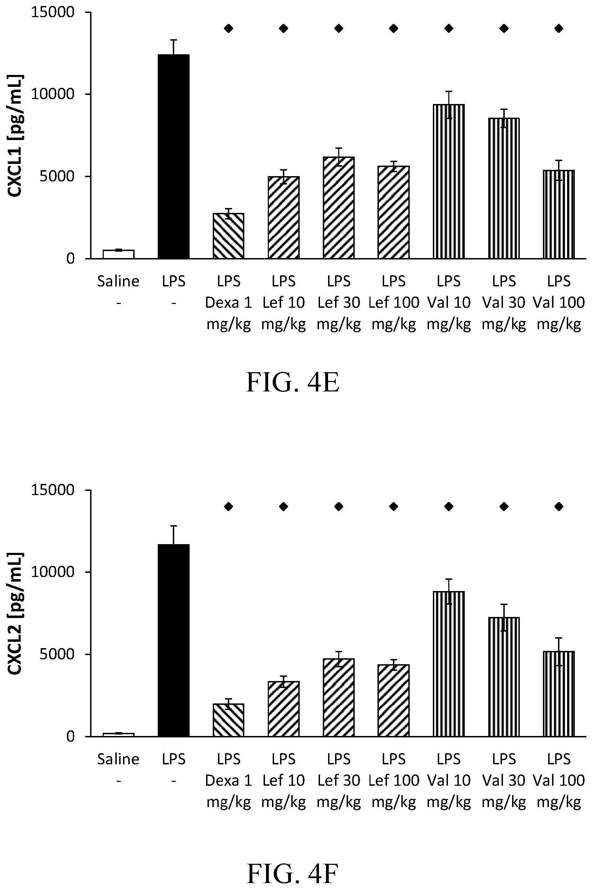

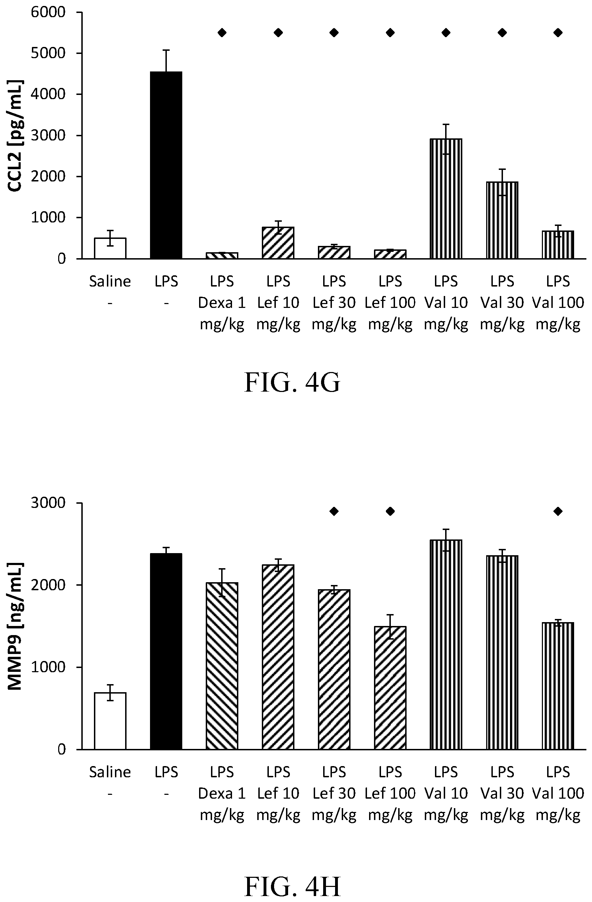

The concentrations of the chemokines (C-X-C motif) ligand 1 (CXCL1), (C-X-C motif) ligand 2 (CXCL2), and (C-C motif) ligand 2 (CCL2) were increased in the lung homogenate after LPS-challenge in the mouse model in comparison to the saline control (Fig. 4E to 4G). Levels of both CXCL1 and CXCL2 were significantly reduced by subcutaneous pretreatment with 10, 30, or 100 mg/kg Lefamulin (Fig. 4E and 4F). At lower doses of 10 and 30 mg/kg Valnemulin showed less effect on CXCL1 and CXCL2 compared to Lefamulin. LPS-induced CCL2 increase in the lung homogenate was completely inhibited at all investigated doses of Lefamulin, whereas Valnemulin was less active particularly at the doses of 10 and 30 mg/kg (Fig. 4G).

The matrix metallopeptidase 9 (MMP9), being a regulatory factor in neutrophil migration, was increased in the LPS-induced mouse model (Fig. 4H). Subcutaneous pre-treatment with 30 or 100 mg/kg Lefamulin reduced MMP9 levels in lung homogenate by 26 or 53%, respectively, whereas it did not alter MMP9 concentrations at 10 mg/kg. Valnemulin reduced the MMP9 concentration only at the highest investigated dose of 100 mg/kg by 50% (Fig. 4H).

In conclusion, this study demonstrated the anti-inflammatory properties of Lefamulin in an LPS-induced lung neutrophilia model in mice by inhibition of neutrophilic infiltration into the lung and by reduction of (pro-)inflammatory factors such as cytokine and chemokine levels in the lung.

The inhibitory potential of Lefamulin in this model is in the same order of magnitude as for the established anti-inflammatory drug Dexamethasone (positive control) with most of the investigated parameters at clinically relevant doses. When compared with Valnemulin at the same doses, Lefamulin surprisingly was shown to be a more potent inhibitor than Valnemulin for most of the (pro-)inflammatory markers.

LPS administration can induce pathologic and biological changes similar to those seen in ARDS or ALI, including neutrophilic infiltration and increased intrapulmonary cytokines, which have been extensively studied in experimental models of acute lung injury (Matute-Bello Gustavo et al.2008, Am J Physiol Lung Cell Mol Physiol 295:L379-399). Further, research has suggested that increased neutrophil recruitment to the lungs may contribute to tissue damage, particularly in chronic diseases or ARDS (Giacalone, Vincent D et al. 2020, Int J Mol Sci 21(3): 851).

The robust anti-inflammatory effects observed already with low doses of Lefamulin (e.g., 10-30 mg/kg) in the described mouse models of neutrophilic inflammation suggest that Lefamulin inhibits either LPS-induced pro-inflammatory signaling, resulting in reduced neutrophil accumulation, or LPS-induced neutrophil infiltration into the lung, resulting in reduced levels of cytokines and chemokines. Both potential mechanisms are consistent with the dose-dependent anti-inflammatory effects observed in vivo following Lefamulin pretreatment.

### Example 3

**Objective:** The example aims at the synthesis of Lefamulin itaconate as a potential new active pharmaceutical ingredient comprising Lefamulin in protonated form as cation and itaconate as an anion derived from the dicarboxylic itaconic acid.

### Methodology and Result:

To a solution of lefamulin as free base (1 g, 1 eq) in DMF (2 mL) itaconic acid was added (0.5 eq) and stirred at room temperature overnight. The resulting reaction mixture was added dropwise to MTBE. The obtained precipitate was filtered, washed with MTBE and dried under reduced pressure to receive Lefamulin itaconate (1.20 g) in the form of a colorless solid.

¹H-NMR (400 MHz, DMSO-d₆, δ, ppm, characteristic signals, mutilin numbering system described in Berner, H.; Schulz, G.; Schneider H. Tetrahedron 1980, 36, 1807-1811): 6.15 (dd, 1 H, H-19, J=17.6, 11.2 Hz), 5.63 (s, 0.5H, itaconate), 5.55 (d, 1H, H-14, J=8.0 Hz), 5.25-5.00 (m, 2.5H, H-20, itaconate), 3.55 and 3.29 (AB, 2H, H-22, J=15.2 Hz), 3.43 (d, 1H, H-11, J=5.6 Hz), 3.05 (s, 1H, itaconate), 1.37 (s, 3H, CH₃-15), 1.06 (s, 3H, CH₃-18), 0.82 (d, 3H, CH₃-17, J=7.2 Hz), 0.63 (d, 3H, CH₃-16, J=6.8 Hz).

MS m/z: 508 [M + H⁺], 552 [M + HCOO⁻].

### Example 4

**Objective:** The anti-inflammatory effects of different salts of Lefamulin were investigated in another modified model of LPS induced lung neutrophilia in analogy to Example 1 with read-out at 24 h post LPS challenge.

**Methodology:** Aqueous solutions of the test compounds were administered as single subcutaneous dose, 30 min prior to intranasal LPS challenge (5 µg/animal). Immediately before the LPS challenge, groups of mice (n=6) were anesthetized via intraperitoneal (IP) injection of ketamine (2 mg/mouse) and xylazine (0.08 mg/mouse). To induce pulmonary neutrophilia, mice in the control group received 50 µL saline and all other animals received 5 µg LPS/50 µL saline intranasally. As control, saline (0.86% NaCl) was applied intranasally as negative control for LPS challenge. Lefamulin was investigated in the form of its acetate salt (Lef.Ac) and in the form of its itaconate salt (Lef.Itacon, synthesized as in Example 3 above), both administered at a dose of 70 mg/kg. For comparison, sodium itaconate (Na.Itacon) was investigated at a dose of 55 mg/kg. The untreated controls received a subcutaneous injection of saline (0.86% NaCl)

Total cell counts and granulocytes cell counts in the BALF were determined 24 hours post intranasal LPS challenge in analogy to Example 1.

### Results:

The results of the BALF analyses (cell counts) are presented in Figure 5. Lefamulin acetate showed a reduction in cell count comparable to the effect shown in previous studies (Example 1 and 2). Sodium itaconate salt by itself already showed some reduction in BALF cell counts.

The properties of itaconic acid and derivatives as immunomodulator have been described before (Yu Xia-Hua et al. 2019, Immunology & Cell Biology 97: 134-141). For example, an ester derivative of itaconic acid (4-Octyl itaconate) showed a protective effect in an LPS induced sepsis model (Liao, Shan-Ting et al. 2019 Nat Comm 10(1); 5091).

The itaconate salt of Lefamulin showed the most pronounced effects on cell counts compared to Lefamulin acetate or Sodium itaconate alone. In this study, Lefamulin itaconate treatment resulted in the lowest total cell counts and granulocyte cell counts and therefore suggest an interesting synergistic or additive effect of the itaconate salt form of Lefamulin.

### Example 5

**Objective:** Effects of Lefamulin were investigated in an *in vivo* Influenza virus model. In the Influenza virus model, mice were challenged with an influenza A (H1N1) strain adapted to mice. The murine immune system is particularly suited for testing the effect on virally induced immune response in a murine viral infection model of influenza.

**Methodology:** Adult female BALB/c mice were randomly allocated to three experimental groups of 15 animals and allowed to acclimatize for one week. Treatments were administered subcutaneously starting at Day -1. The negative control group received a vehicle administered twice per day. Lefamulin was investigated at different dosing. In the low dose regime 35 mg/kg of Lefamulin were administered twice daily (corresponding to a dose of 70 mg/kg/day) from Day -1 to Day 6. In the high dose regime, Lefamulin was administered with a dose of 105/mg/kg/day in three injections until day 3 and was altered from Day 3 on due to administration problems at the injection site. The following doses were 70 mg/kg administered twice daily (corresponding to 140 mg/kg/day). On Day 0, all groups were challenged with influenza A/Puerto Rico/8/34 (H1N1).

During the study, animals were scored daily for clinical signs of influenza virus infection to include abnormal coat condition (piloerection), abnormal posture (hunched), abnormal breathing (rapid and/or irregular breathing rate), reduced mobility, ocular discharged, eye closure and/or survival. The signs of severity of disease were added for the scoring system yielding a maximum possible score of 5. When clinical signs were judged as severe, individual animals were taken out of the study prior to the scheduled end of the study.

At Day 6, lung tissue was dissected, assessed for gross pathology, preserved in fixative and stored for histopathology.

Lung consolidation was scored as follows after macroscopic evaluation: >50% (across all lobes) of field occupied by intra-alveolar edema/haemorrhage; extensive vascular degeneration. Lungs removed and fixated on Day 6 were evaluated microscopically in the histopathology. Four main readouts were evaluated (Bronchial/Bronchiolar Degeneration/Hyperplasia, Broncho-interstitial Inflammation, Alveolar Inflammation/Degeneration, Alveolar Edema/Haemorrhage) and scored yielding a maximum total histopathology score of 16, wherein a lower number indicates less signs of histopathological anomalies.

Lung samples were also processed and stored for Day 3 and Day 6 viral titre. On Day 3 and 6, lungs were collected, homogenised and clarified to determine viral load by TCID₅₀ assay on Madin-Darby Canine Kidney (MDCK) cells.

At Day 3 and 6, bronchoalveolar lavage fluid (BALF) samples were collected and cells processed for flow cytometry analysis of immune cells components using the antibody panel detailed below. Absolute cell counts were performed using flow cytometry counting beads. Cells were gated from viable, single events. Effect of test treatment on immune cell subsets was analysed from the following markers: CD45, TCRβ, CD3, CD4, CD8, CD19, Ly6C, Ly6G, MHCII, CD11b, CD11c, CD49b, Siglec-F, CD64 and a viability dye. Flow cytometry data for Oseltamivir treatment group at Day 3 was not collected due to a processing error.

### Results:

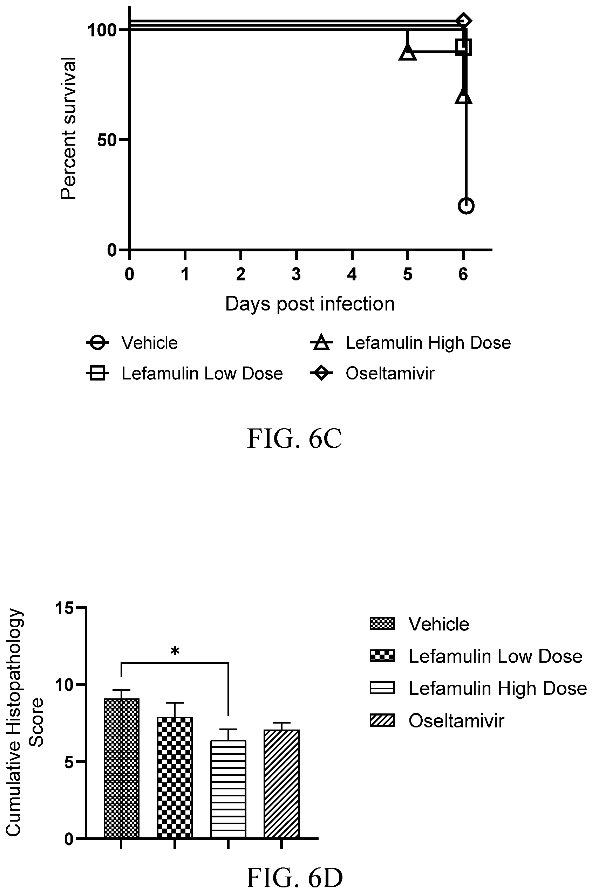

The results of the clinical monitoring are shown in Figures 6A to 6D. The positive control treatment with Oseltamivir worked as expected. A reduction was observed in clinical scores and bodyweight loss for Oseltamivir. Lefamulin did not have a significant effect on bodyweight at the investigated doses (Fig. 6A). At high dose, Lefamulin resulted in an increase in clinical scores and decrease in survival compared to vehicle, which might relate to the local tolerability (SC) issues of the investigated dosage, concentration and formulation (Fig. 6B and C). With the lower dose of Lefamulin, 90 % survival was achieved, whereas only 20 % of the vehicle group survived until day 6 (Fig. 6C).

Moreover, a significant improvement in the macroscopically evaluated lung consolidation was observed for Lefamulin at the low dose. In histopathology, a spectrum of overall individual animal scores (4-13 range) were evident within the specimens examined (Fig. 6D). Lesions were similar to those described within the literature. Increasing severities/distribution area of alveolar pathology were correlated with increases in bronchiolar degeneration/proliferation, broncho-interstitial inflammation and alveola oedema/haemorrhage. Treatment with the high dose of Lefamulin resulted in a significant reduction in bronchial degeneration and alveolar inflammation resulting in an overall significant reduction in histopathology score in this group compared to the vehicle treated control and comparable to Oseltamivir.

Lung viral titre decreased in all groups between Day 3 and Day 6 (Figure 7). Lefamulin at both doses and Oseltamivir resulted in reduced lung viral titres when compared with the vehicle treated control.

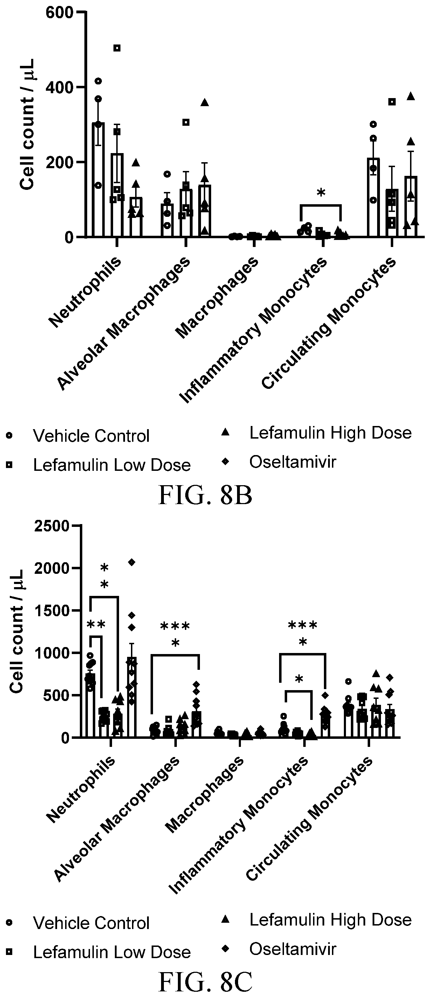

The results of the flow cytometry of BALF are shown in Figures 8A to 8E. The total immune cell count in BALF increased from Day 3 to Day 6 for all study groups (Fig. 8A), which confirms the expected immune cell infiltration induced by the progressing viral infection causing inflammations and cell degeneration in the lung tissue. Lefamulin significantly decreased the total immune cell infiltration in the lung by Day 6 at both doses tested (*p*<0.05). Figures 8B to 8E show the cell count for specific immune cell subsets at Day 3 and 6. Lefamulin significantly decreased neutrophils infiltration at both doses tested by Day 6 (Fig. 8C). Lefamulin when administrated at a higher dose significantly reduced inflammatory monocyte infiltration in the lung at Day 6 after H1N1 challenge (Fig. 8C). Infiltration of NK, CD4 and CD8 cells in the lung was decreased at Day 6 at both doses tested (Fig. 8E). B cells reduction was only significant at the highest dose at Day 6 (Fig. 8E).

The BALF results confirm the immune-modulatory effect of Lefamulin, especially on the neutrophil infiltration into the lung, also in the context of a viral disease model. The clinical readout and the reductive effect on the lung viral titer further supports the potential of Lefamulin in the treatment of viral diseases.

### Example 6

**Objective:** The assay measured the inhibition of virus-induced cytopathic effects (CPE) and cell viability following alpha coronavirus 229E (HCoV-229E or CoV_{229E}) in MRC-5 cells 6 days post infections with the virus by various concentrations of Lefamulin (BC-3781).

**Methodology:** MRC-5 cells were seeded in 96-well flat-bottom tissue culture plates (at a density of 3 x 10³ cells per well) and allowed to adhere overnight. Thereafter, diluted test compounds (Lefamulin as acetate, Tiamulin as fumarate) dissolved in DMSO were added to the plate and incubated for 4 hours prior to addition of the virus. The virus was added diluted to a pre-determined titer to yield 85-95% cell killing at 6 days post-infection (MOI of 0.001).

Following incubation at 37°C and at 5% CO₂ for 6 days, cell viability was measured by XTT tetrazolium dye staining. The optical density of the cell culture plate was determined spectrophotometrically at 450 and 650 nm. Percent reduction of the virus-infected cells and the percent cell viability of uninfected drug control wells were calculated to determine the effective concentration at which 50% of cytopathic effect was inhibited (EC₅₀) and the cytotoxic concentration (TC₅₀) using four parameter curve fit analysis. The antiviral compound Remdesivir served as positive control.

### Results:

Surprisingly, Lefamulin reduced the viral CPE by 91.82% at a concentration of 10 µM, which is a concentration that had no cytotoxic effect on the viability of the cell control. The calculated EC₅₀ was 3.87 µM, at which 50% of the viral cytopathic effect was inhibited. At the Lefamulin concentration of 50 µM, Lefamulin displayed a cytotoxic effect; the calculated TC₅₀ was 55.3 µM. The ratio of EC₅₀ and TC₅₀, known also as therapeutic index, was 14.3.

In contrast, Tiamulin at a concentration of 10 µM reduced the viral CPE only by 10.53% and no cytotoxic effect was observed. At the next higher test concentration of 50 µM the CPE was reduced by 81.68% and a cytotoxic effect was observed. The calculated EC₅₀ was 24.4 µM and the calculated TC₅₀ was 62.9 µM. The therapeutic index of Tiamulin was 2.58 and surprisingly much lower than that of Lefamulin.

The antiviral compound Remdesivir was developed as a treatment for Ebola virus, and also is known to have antiviral activity against corona viruses (clinical investigation is ongoing). Thus, Remdesivir served as positive control herein. Remdesivir showed an EC₅₀ of 0.11 µM, a TC₅₀ of > 5 and a therapeutic index of > 45.5.

| Compound | MRC-5 cells infected with CoV_{229E} | | |
|---|---|---|---|
| | EC₅₀ (µM) | TC₅₀ (µM) | Therapeutic Index |
| Lefamulin | 3.87 | 55.3 | 14.3 |
| Tiamulin | 24.4 | 62.9 | 2.58 |
| Remdesivir | 0.11 | >5.00 | >45.5 |

The results are graphically displayed in Figures 9A (Lefamulin), 9B (Tiamulin) and 9C (Remdesivir) (VC....reduction in viral CPE, CC.....Cell Control).

### Example 7:

**Objective:** The assay measured the inhibition of virus-induced cytopathic effects (CPE) and cell viability following alpha coronavirus 229E (HCoV-229E or CoV_{229E}) in MRC-5 cells for Lefamulin at various treatment conditions.

**Method:** The assay was performed in analogy to Example 6 above with the following differences regarding the test candidate. Lefamulin (as acetate) was evaluated using different treatment conditions of 4 h, 1 h or 0 h incubation prior to virus addition and addition 1 h after infection For this particular set of experiments, coronavirus was diluted 1:200 in assay medium and added at 100 µL/well to achieve approximately 90% cell killing in the untreated virus control wells (MOI of 0.001).

### Results:

The antiviral efficacy and cellular toxicity data are summarized in the table below. Lefamulin showed a time-dependent effect on the inhibition of the virus-induced cytopathic effects (CPE). In the treatment setting after viral exposure (1h post-infection) a dose-dependent effect was observed. At a concentration of 50 µM the viral CPE was reduced by 86.83 % (data not shown in detail).

| Treatment condition | MRC-5 cells infected with CoV_{229E} | | |
|---|---|---|---|
| Lefamulin | EC₅₀ (µM) | TC₅₀ (µM) | Therapeutic Index |
| 4 h pretreatment | 2.18 | >50.0 | > 22.9 |
| 1 h pretreatment | 2.16 | >50.0 | > 23,1 |
| 0 h pretreatment | 3.08 | >50.0 | >16.2 |
| 1 h post-infection | 15.1 | >50.0 | >3.31 |

### Example 8:

**Objective:** The assay measured the inhibition of virus-induced cytopathic effects (CPE) and cell viability following human respiratory syncytial virus (strain RSV_{A2}) replication in HEp2 cells 6 days post infections with the virus by various concentrations of Lefamulin (BC-3781).

**Methodology:** HEp2 cells were seeded in 96-well flat-bottom tissue culture plates (at a density of 5 x 10³ cells per well) and allowed to adhere overnight. Thereafter, diluted test compounds (Lefamulin as acetate, Tiamulin as fumarate) in DMSO were added to the plate and incubated for 4 hours prior to addition of the virus. The virus was added diluted to a pre-determined titer to yield 85-95% cell killing at 6 days post-infection (MOI of 0.001).

Following incubation at 37°C and at 5% CO₂ for 6 days, cell viability was measured by XTT tetrazolium dye staining. The optical density of the cell culture plate was determined spectrophotometrically at 450 and 650 nm. Percent reduction of the virus-infected cells and the percent cell viability of uninfected drug control wells were calculated to determine the effective concentration at which 50% of cytopathic effect was inhibited (EC₅₀) and the cytotoxic concentration (TC₅₀) using four parameter curve fit analysis. The antiviral compound TMC353121 (RSV fusion inhibitor) served as positive control.

### Results:

Surprisingly, Lefamulin reduced the viral cytopathic effect (CPE) by 92.17% and 100% at concentrations of 10 µM and 50 µM, respectively, which are concentrations that had no cytotoxic effect on the viability of the cell control. The calculated EC₅₀ was 5.34 µM, at which 50% of the viral CPE was inhibited. At the Lefamulin concentration of 100 µM, Lefamulin displayed a cytotoxic effect; the calculated TC₅₀ was 70.7 µM. The ratio of EC₅₀ and TC₅₀, known also as therapeutic index, was 13.2.

In contrast, Tiamulin at a concentration of 10 µM reduced the viral CPE only by 16.76% and a cytotoxic effect (84% viability) was observed at this concentration. At the next higher test concentration of 50 µM the viral CPE was reduced by 43.28% and at the cytotoxic effect was more pronounced (70.0% viability). The calculated EC₅₀ was with >67.9 µM above the calculated TC₅₀ of 67.9 µM. The therapeutic index of Tiamulin therefore could not be calculated. Surprisingly, the antiviral activity and the therapeutic index was much higher for Lefamulin than for Tiamulin.

The antiviral compound TMC353121 was developed as a specific respiratory syncytial virus fusion inhibitor (clinical investigation is ongoing). Thus, TMC353121 served as positive control herein. TMC353121 showed an EC₅₀ of 0.006 µM, a TC₅₀ of > 0.1 µM and a therapeutic index of > 167.

| Compound | HEp2 cells infected with human respiratory syncytial virus (RSVS_{A2}) | | |
|---|---|---|---|
| | EC₅₀ (µM) | TC₅₀ (µM) | Therapeutic Index |
| Lefamulin | 5.34 | 70.7 | 13.2 |
| Tiamulin | >67.9 | 67.9 | -- |
| TMC353121 | 0.0006 | >0.1 | >167 |

The results are graphically displayed in Figures 10A (Lefamulin), 10B (Tiamulin) and 10C (TMC353121) (VC....Reduction in viral CPE, CC.....Cell Control).

### Example 9

**Objective:** The assay measured the inhibition of virus-induced cytopathic effects (CPE) and cell viability following human respiratory syncytial virus (strain RSV_{A2}) replication in HEp2 cells changing the multiplicity of infection (MOI).

**Methodology:** The assay was performed in analogy to Example 8 above with the following differences regarding the test. The virus was added diluted to a pre-determined titer to yield 85-95% cell killing at 6 days post-infection, and the added amount was adopted to obtain MOIs of 0.003, 0.001, 0.0008, and 0.0004, respectively. Lefamulin (as acetate) was investigated in this study as well as TMC353121 for positive control.

### Results:

The antiviral efficacy and cellular toxicity data are summarized in the tables below. The EC₅₀ value in the low µM range for Lefamulin was reproduced at an MOI of 0.0004. In contrast, a higher MOI, thus higher viral load with respect to the investigated cells, reduced the antiviral effect of Lefamulin. This effect is less pronounced in the highly effective control substance TMC353121.

| MOI / Compound | HEp2 cells infected with human respiratory syncytial virus (RSV_{A2}) | | |
|---|---|---|---|
| Lefamulin | EC₅₀ (µM) | TC₅₀ (µM) | Therapeutic Index |
| 0.003 | > 63.2 | 63.2 | --- |
| 0.001 | 32.3 | 65.9 | 2.04 |
| 0.0008 | 28.7 | 66.60 | 2.32 |
| 0.0004 | 2.34 | 67.4 | 28.8 |
| | | | |

| MOI / Compound | HEp2 cells infected with human respiratory syncytial virus (RSV_{A2}) | | |
|---|---|---|---|
| TMC353121 | EC₅₀ (µM) | TC₅₀ (µM) | Therapeutic Index |
| 0.003 | 0.0002 | >0.1 | 500 |
| 0.001 | 0.0002 | >0.1 | >500 |
| 0.0008 | 0.0003 | >0.1 | >333 |
| 0.0004 | 0.00004 | >0.1 | >2500 |

### Example 10

**Objective:** The assay measured the inhibition of virus-induced cytopathic effects (CPE) and cell viability following replication of the two different respiratory syncytial virus strains RSV A_{LONG} and RSV B₁₈₅₃₇ in HEp2 cells.

**Methodology:** The assay was performed in analogy to Example 8 above with the difference that cells seeded with a density of 5 x 10³ cells per well were incubated with the virus strains RSV A_{LONG} or RSV B₁₈₅₃₇, respectively, following a 4 hour cell pretreatment with the test compound at different concentrations. Virus was diluted and added in an amount yielding MOIs of 0.01 and 0.001 for RSV A_{LONG} and RSV B₁₈₅₃₇, respectively.

### Results:

The antiviral efficacy and cellular toxicity data are summarized in the tables below. The control compound TMC353121 was evaluated in parallel to Lefamulin and yielded an EC₅₀ value of 0.01 nM against the investigated strains of RSV A and RSV B. Lefamulin yielded an EC₅₀ value of 17.7 µM against the RSV B₁₈₅₃₇. Activity against RSV A_{LONG} could not be determined due to the cytotoxicity to HEp2 cells with TC₅₀ values of 71.1 µM in the assay.

| | HEp2 cells infected with respiratory syncytial virus (RSV A_{LONG}) | | |
|---|---|---|---|
| Compound | EC₅₀ (µM) | TC₅₀ (µM) | Therapeutic Index |
| Lefamulin | >62.9 | 62.9 | --- |
| TMC353121 | 0.00001 | >1.00 | >100000 |
| | | | |

| | HEp2 cells infected with respiratory syncytial virus (RSV B₁₈₅₃₇) | | |
|---|---|---|---|
| Compound | EC₅₀ (µM) | TC₅₀ (µM) | Therapeutic Index |
| Lefamulin | 17.7 | 71.1 | 4.02 |
| TMC353121 | 0.00001 | >1.00 | >100000 |

### Example 11

**Objective:** The assay measured the inhibition of virus-induced cytopathic effects (CPE) and cell viability during replication of Measles virus strain Edmonston in HeLa cells.

**Method:** HeLa cells were seeded in 96-well flat-bottom tissue culture plates (at a density of 5 x 10³ cells per well) and allowed to adhere overnight. Thereafter, diluted test compounds (Lefamulin as acetate, Ribavirin for control) were added to the plate and incubated for 4 hours prior to addition of the virus. Virus was added diluted to a pre-determined titer to yield 85-95% cell killing at 6 days post-infection (1:50 dilution, MOI of 0.008).

Cell viability determination and calculation of EC₅₀ and TC₅₀ were performed as described in Examples 6 and 8.

### Results:

The antiviral efficacy and cellular toxicity data are summarized in the Table below. Ribavirin was evaluated as control compound in parallel to Lefamulin and yielded an EC₅₀ value of 1.88 µg/mL. Surprisingly, Lefamulin yielded an even lower EC₅₀ value of 0.89 µM with a high calculated TI of 81.7.

| Compound | HeLa Cells infected with Measles virus (strain Edmonston) | | |
|---|---|---|---|
| | EC₅₀ (µM) | TC₅₀ (µM) | Therapeutic Index |
| Lefamulin | 0.89 | 72.7 | 81.7 |
| Ribavirin (µg/mL) | 1.88 | 21.6 | 11.5 |

### Example 12

**Objective:** The assay measured the inhibition of virus-induced cytopathic effects (CPE) and cell viability during replication of Dengue virus strain DENV2_{New Guinea} in Huh7 cells.

**Method:** Huh7 cells were seeded in 96-well flat-bottom tissue culture plates (at a density of 5 x 10³ cells per well) and allowed to adhere overnight. Thereafter, diluted test compounds (Lefamulin as acetate, Ribavirin for control) were added to the plate and incubated for 4 hours prior to addition of the virus. The Dengue virus strain DENV2_{New Guinea} was obtained from ATCC (VR-1584) and was grown in Rhesus monkey kidney cells for the production of stock virus pools. Virus was added diluted to a pre-determined titer to yield 85-95% cell killing at 6 days post-infection (MOI of 0.001).

Cell viability determination as well as EC₅₀ and TC₅₀ calculations were performed as described in Examples 6 and 8.

### Results:

The antiviral efficacy and cellular toxicity data are summarized in the Table below. Ribavirin was evaluated as control compound in parallel to Lefamulin and yielded an EC₅₀ value of 4.73 µg/mL. Lefamulin yielded an EC₅₀ value of 6.79 µM. Ribavirin and Lefamulin both showed a certain cytotoxicity for this specific cell line at concentrations of 48.5 µg/mL and 23.3 µM, respectively.

| Compound | Huh7 Cells infected with Dengue virus (strain DENV2_{New Guinea}) | | |
|---|---|---|---|
| | EC₅₀ (µM) | TC₅₀ (µM) | Therapeutic Index |
| Lefamulin | 6.79 | 23.3 | 3.43 |
| Ribavirin (µg/mL) | 4.73 | 48.5 | 10.3 |

### Example 13

**Objective:** The assay measured the inhibition of virus-induced cytopathic effects (CPE) and cell viability during replication of Zika virus strain ZIKV_{PRVABC59} in Huh7 cells following a 4 hour cell pretreatment.

**Method:** Huh7 cells were seeded in 96-well flat-bottom tissue culture plates (at a density of 5 x 10³ cells per well) and allowed to adhere overnight. Thereafter, diluted test compounds (Lefamulin as acetate, Sofosbuvir for control) were added to the plate and incubated for 4 hours prior to addition of the virus. The Zika virus strain PRVABC59 obtained from ATCC (catalog VR-1843) was obtained from ATCC (VR-1584) and was grown in Rhesus monkey kidney cells for the production of stock virus pools. Virus was added diluted to a pre-determined titer to yield 85-95% cell killing at 6 days post-infection (MOI of 0.001).

Cell viability determination as well as EC₅₀ and TC₅₀ calculations were performed as described in Examples 6 and 8.

### Results:

The antiviral efficacy and cellular toxicity data are summarized in the Table below. The control compound Sofosbuvir was evaluated in parallel to Lefamulin and yielded an EC₅₀ value of 0.65 µg/mL. Lefamulin yielded an EC50 value of 2.78 µM with a calculated therapeutic index of 8.42.

| Compound | Huh7 Cells infected with Zika virus (strain ZIKV_{PRVABC59}) | | |
|---|---|---|---|
| | EC₅₀ (µM) | TC₅₀ (µM) | Therapeutic Index |
| Lefamulin | 2.78 | 23.4 | 8.42 |
| Sofosbuvir | 0.65 | > 10 | > 15.4 |

### Example 14

**Objective:** The assay measured the inhibition of virus-induced cytopathic effects (CPE) and cell viability during replication of human rhinovirus strain HRV16 strain 11757 in H1-HeLa cells following a 4 hour cell pretreatment.

**Method:** H1-HeLa cells were seeded in 96-well flat-bottom tissue culture plates (at a density of 5 x 10³ cells per well) and allowed to adhere overnight. Thereafter, diluted test compounds (Lefamulin as acetate, Rupintrivir for control) were added to the plate and incubated for 4 hours prior to addition of the virus. The virus HRV16₁₁₇₅₇ was added diluted to a pre-determined titer to yield 85-95% cell killing in the untreated virus control wells (MOI of 0.0005).

Cell viability determination as well as EC₅₀ and TC₅₀ calculations were performed as described in Examples 6 and 8.

### Results:

Rupintrivir, a protease inhibitor developed for treatment of rhinoviruses, was evaluated in parallel and yielded an EC₅₀ value of 4.90 nM. Lefamulin yielded an EC₅₀ value of 9.34 µM with a calculated therapeutic index of 2.58.

| Compound | MDCK Cells infected with rhinovirus HRV16₁₁₇₅₇ | | |
|---|---|---|---|
| | EC₅₀ (µM) | TC₅₀ (µM) | Therapeutic Index |
| Lefamulin | 9.34 | 24.1 | 2.58 |
| Rupintrivir | 0.0049 | > 0.10 | > 20.4 |

### Example 15

**Objective:** The assay measured the inhibition of virus-induced cytopathic effects (CPE) and cell viability during replication of influenza virus strain A/PR/8/34 in MDCK cells following a 4 hour cell pretreatment.

**Method:** MDCK cells were seeded in 96-well flat-bottom tissue culture plates (at a density of 5 x 10³ cells per well) and allowed to adhere overnight. Thereafter, diluted test compounds (Lefamulin as acetate, Oseltamivir for control) were added to the plate and incubated for 4 hours prior to addition of the virus. The influenza virus strain A/PR/8/34 was added diluted to a pre-determined titer to yield 90% cell killing in the untreated virus control wells (MOI of 0.0004).

Cell viability determination as well as EC₅₀ and TC₅₀ calculations were performed as described in Examples 6 and 8.

### Results:

Oseltamivir as established influenza drug was evaluated in parallel and yielded an EC₅₀ value of 0.06 µM. Lefamulin was cytotoxic to MDCK cells at concentrations greater than 23 µM. A maximum inhibition of the influenza mediated CPE by 18.4% was measured at 5 µM Lefamulin. Therefore, and because cytotoxicity was observed at 50 µM, an EC₅₀ could not be determined for Lefamulin. Of note, an *in vivo* activity was observed in an Influenza infection mouse model (see Example 5 above) using a related mouse adapted Influenza A strain (Influenza A/Puerto Rico/8/34 (H1N1)).

| Compound | MDCK Cells infected with influenza virus (strain A/PR/8/34) | | |
|---|---|---|---|
| | EC₅₀ (µM) | TC₅₀ (µM) | Therapeutic Index |
| Lefamulin | > 23.8 | 23.8 | --- |
| Oseltamivir | 0.06 | > 200 | > 333 |

## Claims

1. Lefamulin or a pharmaceutically acceptable salt, solvate, or prodrug thereof for the specific use in the treatment or prevention of a neutrophilic inflammation.

2. Lefamulin or a pharmaceutically acceptable salt, solvate, or prodrug thereof for use according to claim 1, wherein Lefamulin reduces the levels of the proinflammatory mediators selected from cytokines, chemokines, or metalloproteinases.

3. Lefamulin or a pharmaceutically acceptable salt, solvate, or prodrug thereof for use according to claim 2, wherein the proinflammatory mediators are selected from from TNF-α, IL-6, IL-1β, GM-CSF, CXCL1, CXCL2, CCL2, or MMP-9.

4. Lefamulin or a pharmaceutically acceptable salt, solvate, or prodrug thereof for use according to any one of claims 1 to 3, wherein the disease is COPD, cystic fibrosis, bronchiectasis, diffuse panbronchiolitis, bronchiolitis obliterans syndrome, or non-eosinophilic asthma.

5. Lefamulin or a pharmaceutically acceptable salt, solvate, or prodrug thereof for use according to any one of claims 1 to 4, wherein the lefamulin or pharmaceutically acceptable salt, solvate, or prodrug thereof is administered by inhalation, by injection, or orally.

6. Lefamulin or a pharmaceutically acceptable salt, solvate, or prodrug thereof for use according to any one of claims 1 to 5, wherein the lefamulin or pharmaceutically acceptable salt, solvate, or prodrug thereof is administered by oral administration.

7. Lefamulin or a pharmaceutically acceptable salt, solvate, or prodrug thereof for use according to of claim 6, wherein the lefamulin or pharmaceutically acceptable salt, solvate, or prodrug thereof is administered in the form of a tablet or a capsule.

8. Lefamulin or a pharmaceutically acceptable salt, solvate, or prodrug thereof for use according to any one of claims 1 to 7, wherein the lefamulin or pharmaceutically acceptable salt, solvate, or prodrug thereof is administered in a pharmaceutical composition or unit dose form comprising about 600 mg of lefamulin or about 600 mg of lefamulin in the form of a pharmaceutically acceptable salt, solvate, or prodrug thereof.

9. Lefamulin or a pharmaceutically acceptable salt, solvate, or prodrug thereof for use according to any one of claims 1 to 8, wherein the disease is not mediated by bacteria.

10. A pharmaceutical composition, wherein a unit dose form of the composition comprises about 600 mg of lefamulin or about 600 mg of lefamulin in the form of a pharmaceutically acceptable salt, solvate, or prodrug thereof and is for oral administration.

11. The pharmaceutical composition of claim 10, wherein the unit dose form is in the form of a tablet or a capsule.
